(19) Europäisches Patentamt / European Patent Office / Office européen des brevets

(11) **EP 3 758 660 B1**

(12) **EUROPÄISCHE PATENTSCHRIFT**

(45) Veröffentlichungstag und Bekanntmachung des Hinweises auf die Patenterteilung:
**01.11.2023   Patentblatt 2023/44**

(21) Anmeldenummer: **19708041.9**

(22) Anmeldetag: **22.02.2019**

(51) Internationale Patentklassifikation (IPC):
**A61F 13/49** (2006.01)     **B32B 5/02** (2006.01)

(52) Gemeinsame Patentklassifikation (CPC):
**A61F 13/4902; A61F 13/49012;** A61F 2013/49022

(86) Internationale Anmeldenummer:
**PCT/EP2019/054416**

(87) Internationale Veröffentlichungsnummer:
**WO 2019/166338 (06.09.2019 Gazette 2019/36)**

(54) **INKONTINENZWEGWERFWINDEL MIT SEITENABSCHNITTEN UND ELASTISCHER KOMPONENTE**

DISPOSABLE INCONTINENCE NAPPY HAVING LATERAL SECTIONS AND ELASTIC COMPONENT

COUCHE JETABLE POUR INCONTINENCE DOTÉE DE SECTIONS LATÉRALES ET D'UN ÉLÉMENT ÉLASTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.02.2018   DE 102018104539**

(43) Veröffentlichungstag der Anmeldung:
**06.01.2021   Patentblatt 2021/01**

(73) Patentinhaber: **Paul Hartmann AG**
**89522 Heidenheim (DE)**

(72) Erfinder:
• **GAUSE, Enno**
**89522 Heidenheim (DE)**
• **SWEREV, Maximilian**
**86169 Augsburg (DE)**
• **KESSELMEIER, Rüdiger**
**89233 Burlafingen (DE)**
• **REISEL, Robert**
**LE10 0YJ Hinckley, Leicestershire (GB)**
• **BENNING, Heiner**
**89537 Giengen (DE)**
• **BAIER, René**
**89527 Illertissen (DE)**
• **WAGNER, Maximilian**
**96047 Bamberg (DE)**
• **KEILHOLZ, Clemens**
**91356 Kirchehrenbach (DE)**
• **SCHMIDT, Dominik**
**90419 Nürnberg (DE)**

(56) Entgegenhaltungen:
| | |
|---|---|
| EP-A1- 1 686 209 | EP-A1- 2 602 282 |
| EP-A1- 3 187 333 | EP-A1- 3 228 291 |
| CA-A1- 2 654 750 | DE-A1-102008 019 030 |
| DE-A1-102014 110 941 | DE-A1-102015 226 815 |
| US-A1- 2002 056 510 | US-A1- 2004 121 692 |
| US-A1- 2005 287 892 | US-A1- 2007 287 348 |
| US-A1- 2011 160 687 | |

EP 3 758 660 B1

**Beschreibung**

Die Erfindung betrifft eine Inkontinenzwegwerfwindel mit einer Längsrichtung und einer Querrichtung und mit einem einen Saugkörper aufweisenden Hauptteil umfassend einen Vorderbereich mit vorderen seitlichen Längsrändern und einen Rückenbereich mit hinteren seitlichen Längsrändern und einen dazwischen angeordneten, zwischen den Beinen eines Benutzers zu liegen kommenden Schrittbereich und mit beidseits an den Rückenbereich angefügten hinteren Seitenabschnitten, welche sich in der Querrichtung über den jeweiligen seitlichen Längsrand des Hauptteils hinauserstrecken. Die Inkontinenzwegwerfwindel ist als mit Verschlusselementen versehene offene Windel (tape-type) vorgesehen.

Dieser Windel-Typ wird, im Gegensatz zu pant-type Windeln, üblicherweise derart an den Körper angelegt, dass Seitenbereiche eines hinteren Teils der Windel in je nach anatomischen Gegebenheiten unterschiedlich starker Überlappung mit der Außenseite eines vorderen Bereichs der Windel gebracht und dort lösbar insbesondere mittels klebenden oder mechanischen Verschlusselementen angeheftet werden. Insbesondere die hinteren Seitenabschnitte weisen im Bereich ihres in Querrichtung freien Endes jeweils wenigstens ein Verschlussmittel auf.

An den hinteren Seitenabschnitten ist eine in der Querrichtung (Hüftumfangsrichtung) elastische Komponente angeordnet, welche elastische Bereiche bildet. Dieser Typ Inkontinenzwegwerfwindel wird nachfolgende auch kurz als "Tape-type-Windel mit Seitenabschnitten" bezeichnet. Eine derartige Tape-type-Windel mit Seitenabschnitten ist beispielsweise in WO 2007042084 A1 offenbart, wobei diese Windel hintere und vordere Seitenabschnitte aufweist. In WO2017114695A1 ist eine derartige Windel als sogenannte T-Form Windel offenbart, das heißt mit lediglich hinteren Seitenabschnitten.

Ein Problem stellt in diesem Zusammenhang die Tatsache dar, dass die entsprechenden Inkontinenzwegwerfwindeln bei bestimmungsgemäßer Verwendung häufig mit Körperpflegemitteln in Kontakt kommen, welche eine ölige Phase enthalten oder gar vollständig auf einer öligen Trägerbasis beruhen (Körperöle). Hierbei werden insbesondere diejenigen Bestandteile eines Verbundmaterials einer elastischen Komponente, welche aus elastomeren Polymermaterialien bestehen, einer zusätzlichen Belastung ausgesetzt, denn die Beständigkeit gegenüber öligen Komponenten ist bei verschiedenen Elastomeren sehr unterschiedlich ausgebildet. Ölige Komponenten (bzw. die öligen Bestandteile der oben genannten Körperpflegemittel) können offenbar dessen mechanische Eigenschaften ungünstig beeinflussen; im ungünstigsten Fall wird dabei die aus dem Verbundmaterial bestehende oder die dieses Verbundmaterial umfassende Windelkomponente zerstört oder verändert dabei in nachteiliger Weise seine Eigenschaften.

Im Stand der Technik sind verschiedene Wege vorgeschlagen, elastische Komponenten gegenüber dem Einfluss öliger Komponenten beständig zu machen.

In der WO 2004/060665 wird vorgeschlagen, eine zentrale elastomere Folienlage beidseitig durch Verklebung mit einem geeigneten Klebstoff mit dem Vliesstoff zu verbinden, wobei der Klebstoff gegen ölige Flüssigkeiten widerstandsfähig ist. Es resultiert dabei ein Verbundmaterial mit insgesamt fünf Lagen (Vliesstoff-Klebstoff-Elastomerfolie-Klebstoff-Vliesstoff), wobei die Klebstofflagen für die Widerstandsfähigkeit gegenüber öligen Flüssigkeiten sorgen. Nachteilig ist hierbei der aufwendige Herstellprozess des Laminates, welcher die Applikation zweier Klebstofflagen erfordert.

In EP 3 187 333 A wird ein Laminat mit zwei äußeren Vliesschichten und einer zentralen Elastomer-Lage beschrieben, wobei die Elastomer-Lage selbst aus mindestens zwei Schichten besteht, wobei mindestens eine Schicht ein olefin-basiertes Elastomer darstellt. Nachteilig ist hier der technologische Aufwand im Zusammenhang mit dem Erfordernis der Bereitstellung einer mehrschichtigen Elastomer-Lage.

US 2004/0121692 A1 offenbart ein kleberverbundenes Folien/Vlies-Laminat.

Bei den Windeln, bei denen die hinteren Seitenabschnitte auf die Außenseite des Vorderbereichs des Windelhauptteils oder ggf. auf die Außenseite der Seitenabschnitte des Vorderbereichs geschlossen werden, werden beim Schließen häufig starke Zugkräfte in die hinteren Seitenabschnitte eingeleitet, weil der Benutzer oder eine Pflegeperson beim Anlegen der Windel bestrebt ist, eine deutliche Überlappungssituation zwischen den hinteren Seitenabschnitten und dem Vorderbereich des Hauptteils herzustellen und hierbei gleichzeitig die für einen Festsitz der Windel erforderliche Zugkraft in das Gesamtsystem einzubringen, so dass die Windel auch dauerhaft am Benutzer gehalten wird.

Es kommt dort daher häufig zum Einreißen der Seitenabschnitte oder zum Auftrennen der Fügeverbindung. Des Weiteren kann es vorkommen, dass die Windel vom Träger der Windel als zu eng und zu fest angelegt empfunden wird. Nicht zuletzt besteht die Gefahr, dass die in der Querrichtung elastische Komponente der hinteren Seitenabschnitte dabei stark einschnürt.

Die vorliegende Erfindung stellt eine Inkontinenzwegwerfwindel bereit mit einer Längsrichtung und einer Querrichtung und mit einem einen Saugkörper aufweisenden Hauptteil umfassend einen Vorderbereich mit vorderen seitlichen Längsrändern und einen Rückenbereich mit hinteren seitlichen Längsrändern und einen dazwischen angeordneten, zwischen den Beinen eines Benutzers zu liegen kommenden Schrittbereich und mit beidseits an den Rückbereich angefügten hinteren Seitenabschnitten, welche sich in der Querrichtung über die hinteren seitlichen Längsränder des Hauptteils hinauserstrecken, und wobei die hinteren Seitenabschnitte je eine in der Querrichtung elastische Komponente aufweisen, wobei die elastische Komponente einen elastischen Verbundwerkstoff umfasst, wobei der Verbundwerkstoff eine elastische Folienlage und eine mit der elastischen Folienlage verbundene erste Vliesstofflage umfasst, wobei die Folienlage einen einschichtigen Aufbau aufweist, wobei die erste Vliesstofflage direkt mit der elastischen Folienlage verbunden ist, wobei der Verbundwerkstoff bei erstmaliger Dehnung in der Querrichtung um 140% eine Rückstellkraft von K140%,1 ≤ 12 N/25mm aufweist und wobei der Verbundwerkstoff eine Glyceryl trioleat Resistenz RGly150% von ≥ 50% aufweist und wobei K140%,1 und RGly150% nach der weiter unten in der Beschreibung jeweils dargelegten Prüfmethode zu ermitteln sind.

Die Glyceryl trioleat Resistenz RGly150% (ermittelt nach der weiter unten beschriebenen Prüfmethode) hat sich als guter Indikator für die Beständigkeit des Verbundwerkstoffes gegenüber handelsüblichen Hautpflegemitteln erwiesen. Mit einer Rückstellkraft von K140%,1 ≤ 12 N/25mm ist sichergestellt, dass die elastische Komponente in Gebrauch der Inkontinenzwegwerfwindel keine einschnürende, den Träger der Windel unangenehm belastende Wirkungen entfaltet.

Nach einer bevorzugten Variante der vorliegenden Erfindung weist der Verbundwerkstoff nach der weiter unten dargelegten Prüfmethode eine Glyceryl trioleat Resistenz RGly150% von ≥ 55%, insbesondere von ≥ 60%, weiter insbesondere von ≥ 65% auf.

Die Glyceryl trioleat Resistenz RGly150% ist weiter bevorzugt ≤ 95%, insbesondere ≤ 90%, weiter insbesondere ≤ 85%.

Die Glyceryl trioleat Resistenz in [%] gibt wie unten noch näher erläutert, den Anteil der Rückstellkraft an, den der Verbundwerkstoff nach (trotz) Einwirken von Glyceryl trioleat bei definierter Dehnung beizubehalten in der Lage ist. Die Resistenz des Verbundwerkstoffs gegenüber Glyceryl trioleat hat sich als weitgehend zuverlässiger Indikator hinsichtlich der Unempfindlichkeit des Verbundwerkstoffes gegenüber handelsüblichen Pflegemitteln herausgestellt.

Bevorzugt weist der Verbundwerkstoff nach der weiter unten dargelegten Prüfmethode eine Rückstellkraft KGly150% (bei Dehnung um 150%) von 2,5 - 12,5 N/40mm, insbesondere von 5,5 - 10,5 N/40mm auf.

Vorteilhafterweise beträgt die Rückstellkraft KGly140% (bei Dehnung um 140%) 1,5 - 7,0 N/40mm, insbesondere 3,0 - 6,0 N/40mm, nach der weiter unten dargelegten Prüfmethode.

Prüfmethode Glyceryl trioleat Resistenz:

Zur Bestimmung der Glyceryl trioleat Resistenz wird das zu prüfende Material unter definierten Bedingungen gedehnt und dem Einwirken von Glyceryl trioleat (CAS Nummer 122-32-7, Reinheit ≥ 99%) ausgesetzt und es werden bei definierten Dehnungen vor und nach dem Einwirken des Glyceryl trioleat die Rückstellkräfte gemessen, die das gedehnte Material der Dehnung entgegensetzt.

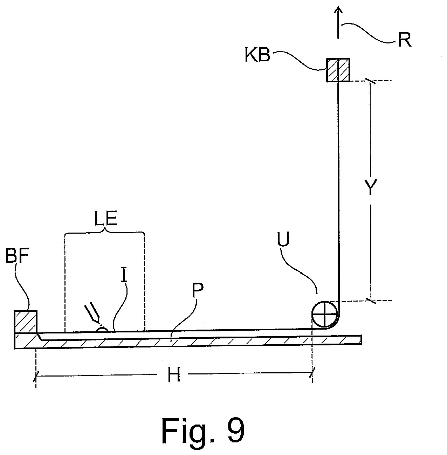

Für die Durchführung der Prüfungen wurde eine Zugprüfmaschine Typ Shimadzu AG-Xplus, gemäß DIN EN ISO 7500-01:2004-11, mit Einbauelement umfassend eine horizontal fixierte Platte P mit feststehender Klemmbacke BF mit einer Klemmbackenbreite von mindestens 50mm und eine Umlenkrolle U (ebenfalls mindestens 50 mm breit; Durchmesser: 22 mm; Lager: E2.626-2Z/C3) verwendet (Figure 9). Vorzugsweise verfügt die Testvorrichtung über eine Software, welche den Prüfzyklus steuert und ein automatisiertes Aufzeichnen der Kraftwerte sichergestellt.

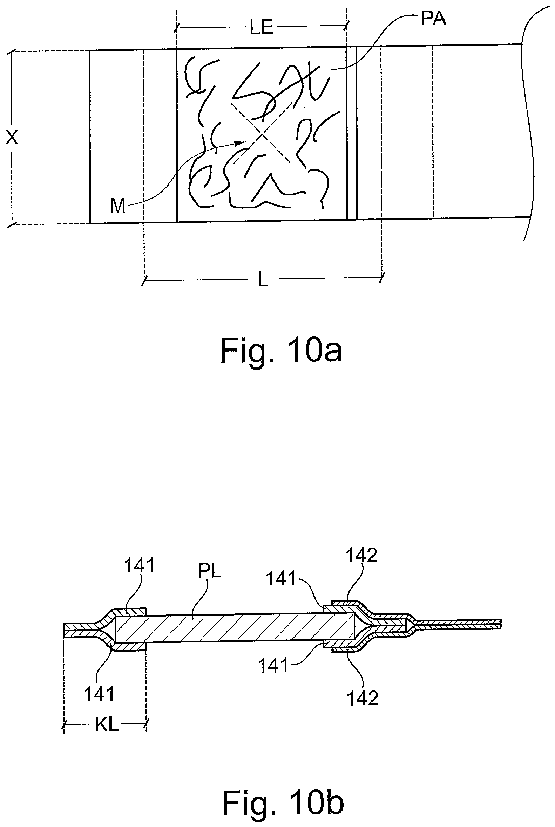

Bei der Durchführung der Prüfmethode wird der zu prüfende Materialabschnitt des elastischen Verbundwerkstoffs einer Breite X von 40 mm und einer Länge L (deren Richtung der Zugrichtung entspricht) von 60 mm bereitgestellt (Figur 10a). Hierzu wird der Prüfling zum Beispiel aus einer entsprechenden elastischen Komponente der Inkontinenzwegwerfwindel herausgestanzt, wobei die Zugrichtung des Prüflings der Querrichtung der Inkontinenzwegwerfwindel entspricht. Zur sicheren Verbindung des Prüflings mit den Klemmbacken des Zugprüfgeräts und der Bereitstellung eines effektiven Prüfabschnittes PA mit einer Länge LE von 40 mm werden die beiden Querenden des Prüflings über ihre volle Breite zunächst durch zwei mit ihren Klebeflächen gegeneinander geklebte einseitige Klebebänder 141 einer Länge KL von 20 mm derart fixiert, dass das obere Klebeband die Außenseite des Prüflings und das untere Klebeband die Innenseite des Prüflings je über eine Länge von 10 mm überfängt (als Klebeband z. Bsp. Filament Tape 8959 fadenverstärkt, bei der Firma 3M Deutschland GmbH, ansässig in Neuss, erhältlich) und dadurch verstärkt und je Seite um ca. 10 mm verlängert (Figuren 10a und 10b). Das Überfangen mit dem steifen, festen Klebeband um je 10mm auf jeder Seite nimmt dem Materialabschnitt in diesem Bereich die Elastizität und verringert dadurch die Länge LE des effektiven Prüfabschnitts PA des Prüflings auf 40 mm (60mm minus 2x10mm). Anschließend erfolgt eine weitere, jedoch einseitige Verlängerung des Prüflings um ca. 30 cm, derart, dass zwei weitere einseitige, steife Klebebänder 142, des oben spezifizierten Typs, einer Breite, die der Breite des Prüflings entspricht, mit ihren Klebeflächen gegeneinander geklebt werden, wobei das untere Klebeband die Rückseite und das obere Klebeband die Oberseite des mit den ersten Klebebändern verstärkten Prüflings um 10-20 mm überfangen (Figur 10a und 10b). Es versteht sich, dass die Klebebandmaterialien so gewählt sind, dass sie eine feste, durch die Beanspruchung während der Prüfung nicht beeinträchtigbare Verbindung mit dem Materialabschnitt eingehen und von ausreichender Steifheit und Festigkeit sind, so dass sie die Zugkräfte vollständig auf den Materialabschnitt übertragen und im für die Prüfung relevanten Kraftbereich selbst keine Dehnung während der Zugprüfung erfahren.

Probenvorbereitung:

Die zu verwendende Komponente, mithin der zu prüfende Materialabschnitt des elastischen Verbundwerkstoffs und das einseitige Klebeband werden über 24 h bei 23° C und 50 % relativer Luftfeuchte konditioniert. Das Glyceryl trioleat weist eine Temperatur von 23°C auf.

Prüfverfahren:

Die Prüfung findet bei 23° C und 50 % relativer Luftfeuchte statt. Das untere Ende des Prüflings wird durch die feststehende Klemmbacke BF des Probentisches P mittig zentriert fixiert, dann über den Probentisch geführt und an der Umlenkrolle U, deren Durchmesser 22 mm beträgt, um 90° nach oben umgelenkt und das gegenüberliegende Ende in die obere bewegliche Klemmbacke KB ebenfalls mittig zentriert spannungsfrei fixiert (Figur 9).

Der so eingespannte Prüfling ist mitsamt der schematisch angedeuteten Zugvorrichtung mit Probentisch P und Umlenkrolle U in Figur 9 dargestellt. Man erkennt den Probentisch P aus poliertem Stahl und die untere Klemmbacke BF. Die Umlenkrolle U ist in horizontalem Abstand H von 145 mm von der unteren Klemmbacke BF angeordnet. Die obere, bewegliche Klemmbacke KB ist im senkrechten Abstand Y von 144 mm von der Umlenkrolle angeordnet. Der Prüfling PL mit dem effektiven Prüfabschnitt PA ist derart orientiert, dass die Innenseite I zuoberst liegt. Die Innenseite ist diejenige Seite, die bestimmt ist, in Gebrauch der Inkontinenzwindel dem Körper des Trägers zugewandt zu sein.

Es wird dann die bewegliche Klemmbacke KB in Pfeilrichtung R bis zu einer Vorkraft von 0,2 N bewegt. Dann wird der Prüfling um 150 % (bezogen auf die Ausgangslänge des effektiven Prüfabschnitts PA nach Anfahren der Vorkraft), mit Prüfgeschwindigkeit von 500mm/min gedehnt und die Kraft bei 150% Dehnung notiert (= K150%) und sodann ohne Haltezeit auf eine Dehnung von 140% durch Zurückfahren der Klemmbacke KB entgegengesetzt zur Pfeilrichtung R entspannt. Nach einer Haltezeit von 10 sec bei 140% Dehnung werden mit einer Pipette 200$\mu$l der Testsubstanz Glyceryl trioleat mittig (Mittelpunkt M, Figur 10a; schematisch angedeutet auch in Figur 9, ermittelt als der Schnittpunkt der Diagonalen des effektiven Prüfabschnitts PA bei Dehnung um 140%) auf die Innenseite I des Prüfabschnitts PA aufgebracht. Nach einer Haltezeit von 180 sec nach Aufgabe der Testsubstanz wird die Kraft notiert (=KGly140%) und es erfolgt eine erneute Dehnung bis auf 150% und die Kraft bei 150% Dehnung (=KGly150%) wird notiert.

Prüfgeschwindigkeit (Be- und Entlastung): 500 mm/min.

Auswertung: Es werden angegeben:

Glyceryl trioleat Resistenz RGly150% in [%] = (KGly150% / K150%) x 100 [%]
Rückstellkraft KGly140% in N/40mm
Rückstellkraft KGly150% in N/40mm

Sollte kein 40 mm breiter Abschnitt der elastischen Komponente zur Verfügung stehen, wird ein entsprechend schmalerer Abschnitt verwendet. Solchenfalls wird die Menge der Testsubstanz Glyceryl trioleat in $\mu$l um den Faktor f reduziert. Zudem werden die ermittelten Kraftwerte rechnerisch auf einen 40 mm breiten Abschnitt normiert, derart dass die gemessenen Kräfte mit demselben Faktor f multipliziert werden, welcher sich ergibt aus f=40/x, wobei x die Breite des Prüflings gemessen in mm ist.

Im Falle eines 20 mm breiten Prüflings beträgt der Faktor f mithin 2. Es würden solchenfalls 100 $\mu$l Glyceryl trioleat aufgebracht werden.

Sollte kein 60 mm langer Abschnitt (in Querrichtung der Inkontinenzwegwerfwindel) der elastischen Komponente zur Verfügung stehen, wird ein entsprechend kürzerer Abschnitt verwendet. Die Länge des effektiven Prüfabschnitts wird ggf. auf bis zu 15 mm verringert.

Nach einer weiteren bevorzugten Ausführungsform der vorliegenden Erfindung ist der Verbundwerkstoff mandelöl- und/oder paraffinöl- und/oder rapsölbeständig wobei diese Eigenschaften wie folgt zu ermitteln sind:

Testmethode Beständigkeit gegenüber Mandelöl oder Paraffinöl oder Rapsöl

Ein quadratischer Prüfabschnitt des elastischen Verbundwerkstoffs einer Größe von 70 mal 70 mm wird an zwei gegenüberliegenden Seiten jeweils in eine Metallschiene fest eingespannt und durch senkrechten Zug um 100 %, d.h., auf eine Länge in Zugrichtung von 140 mm, gedehnt. Die Zugrichtung entspricht hierbei der Querrichtung der Inkontinenzwindel. Auf den gespannten Prüfabschnitt wird mittels einer Pipette mittig ein Tropfen der Prüfflüssigkeit mit 0,030 g aufgebracht. In einem Zeitraum von drei Stunden wird der Prüfabschnitt wiederholt visuell kontrolliert. Werden nach drei Stunden keine mechanischen Veränderungen (Löcher, Risse und dergleichen) an der Auftragungsstelle bzw. um die Auftragungsstelle herum festgestellt, gilt das Material gemäß der hier verwendeten Definition als beständig gegenüber der jeweiligen Komponente Mandelöl oder Rapsöl oder Paraffinöl.

Als standardisierte Prüfflüssigkeiten werden die in Hautpflegemitteln häufig verwendeten Öle Mandelöl (mit der CAS-Nr. 90320-37-9, einer relativen Dichte von 0,916 ( 20°C ), einer Säurezahl von $\leq$ 1,0 mg KOH/g und einer Peroxidzahl $\leq$ 5), Paraffinöl (der Firma VWR Chemicals mit der Ref.Nr. 301440ZK) oder Rapsöl (CAS-Nr. 8002-13-9, relative Dichte von 0,917 (20°C), Säurezahl von maximal 0,5 mg KOH/g, Peroxidzahl von maximal 10 meq O2/kg) verwendet.

Sollte kein 70x70 mm großer Prüfabschnitt zur Verfügung stehen, wird ein entsprechend kleinerer Abschnitt gewählt.

Nach einer bevorzugten Ausführungsform enthält die elastische Folienlage weniger als 10 Gew.-% medizinische Weißöle, insbesondere weniger als 5 Gew.-% , weiter insbesondere weniger als 3 Gew.-%, weiter insbesondere keine (0 Gew.-%) medizinischen Weißöle.

Nach einer weiteren bevorzugten Ausführungsform enthält die elastische Folienlage weniger als 10 Gew-% technische Weißöle, insbesondere weniger als 5 Gew.-%, insbesondere weniger als 3 Gew.-%, weiter insbesondere keine (0 Gew.-%) technischen Weißöle.

Insbesondere beträgt die Summe der technischen und medizinischen Weißöle weniger als 10 Gew.-%, insbesondere weniger als 5 Gew.-%, insbesondere weniger als 3 Gew.-%, insbesondere 0 Gew.-% der elastischen Folienlage.

Nach einer weiteren Ausführungsform enthält die elastische Folienlage weniger als 10 Gew.-%, insbesondere weniger als 5 Gew.-%, insbesondere weniger als 3 Gew.-% insbesondere keine (also 0 Gew.-%) Mineralöle.

Nach einer weiteren Ausführungsform enthält die elastische Folienlage weniger als 10 Gew.-%, insbesondere weniger als 5 Gew.-%, insbesondere weniger als 3 Gew.-%, weiter insbesondere keine (also 0 Gew.-%) Weichmacher.

Weichmacher oder sogenannte "Plastiziser" dienen im Allgemeinen dazu, Kunststoffe weicher und flexibler zu machen. Alle im Stand der Technik zu diesem Zweck verwendeten und dem Fachmann bekannten weichmachenden Substanzen können unter dem Begriff "Weichmacher" im Sinne dieser Beschreibung verstanden werden. Dazu gehören unter anderem Poly-Alpha-Olefine (z.B. amorphe Polyalphaolefine ), (funktionalisierte) Oligomere wie Oligobutadien, -isoprene, flüssige Nitrilkautschuke, flüssige Terpenharze, pflanzliche und tierische Öle und Fette, Esterweichmacher, wie z.B. Phtalate, Adipate, Mellitate, phosphorhaltige Ester oder funktionalisierte Acrylate. Zu der Gruppe der Weichmacher gehören ebenso Mineralöle, die paraffinische, aromatische oder naphthenische Bestandteile enthalten.

Weißöle sind Paraffinöle und gehören je nach Herstellungsverfahren zu den Mineralölen oder den synthetischen Ölen. Technische Weißöle, liegen kaum oder nur einmal hydriert vor und können noch Spuren von aromatischen Kohlenwasserstoffverbindungen enthalten (z.B. Flavex der Firma Shell).

Medizinische Weißöle, sind hochgereinigte, also zum Beispiel mehrfach raffinierte Paraffinöle ohne Rückstände wie aromatische Kohlenwasserstoffe und Schwefelverbindungen. (z.B. Ondina X der Fa. Shell ). Medizinische Weißöle werden deshalb in Produkten eingesetzt, die hohe Reinheitsgrade ohne Verschmutzung durch gesundheitlich bedenkliche Substanzen wie aromatische Kohlenwasserstoffe etc. aufweisen müssen, wie z.B. in der Kosmetik-, Medizinprodukte- oder Hygieneindustrie. Sie erfüllen die Anforderungen des Europäischem Arzneibuchs (3. Ausgabe 1997, Paraffinum liquidum), wobei sie abweichend davon auch synthetischen Ursprungs sein können.

Handelsübliche Pflegemittel weisen häufig eine Vielzahl an öligen, teils synthetischen teils aus Naturstoffen gewonnenen Komponenten auf. Ohne an eine Theorie gebunden zu sein, wird angenommen, dass diese Komponenten handelsüblicher Pflegemittel in der Lage sind, entsprechende Bestandteile des Kunststoffes der elastischen Folienlage zu lösen und damit zu schädigen. Die Begrenzung des Anteils von medizinischen Weißölen, insbesondere technischen Weißölen, weiter insbesondere Mineralölen und besonders bevorzugt von sämtlichen Weichmachern in der elastischen Folienlage kann wohl deshalb dazu beitragen, das Ausmaß der schädigenden Wirkung handelsüblicher Pflegemittel zu begrenzen.

Nach einer weiteren Variante ist die elastische Komponente in der Längsrichtung der Inkontinenzwegwerfwindel nicht elastisch, insbesondere undehnbar ausgebildet. Vorzugsweise ist vorgesehen, dass die elastische Folienlage eine Polymermischung umfasst oder daraus besteht. Besonders bevorzugt enthält die Polymermischung mindestens ein thermoplastisches Elastomer aus der Gruppe der Styrol/Isopren/Styrol-Triblock-Copolymere (SIS). Ferner enthält die Polymermischung vorzugsweise mindestens ein Polymer, ausgewählt aus der Gruppe der olefinischen Polymere, wobei die olefinischen Polymere vorzugsweise ausgewählt sind aus der Gruppe der Polyethylene und Polypropylene und Copolymere von Ethylen und Propylen und thermoplastischen elastomeren Polyolefinen (TPOs).

In einer bevorzugten Ausführungsform sind die olefinischen Polymere der Polymermischung der elastischen Folienlage ausgewählt aus der Gruppe der mittels Metallocen-Katalyse hergestellten Polyethylene und Polypropylene und Copolymeren von Ethylen und Propylen. In einer weiteren bevorzugten Ausführungsform ist das olefinische Polymer der Polymermischung ein Polypropylen oder ein Copolymer von Ethylen und Propylen.

Die elastische Folienlage weist einen einschichtigen Aufbau auf.

Die erste Vliesstofflage ist direkt, das heißt ohne Verwendung eines separaten Klebstoffes/Bindemittels/Haftvermittlers mit der Folienlage verbunden ist. Vorzugsweise ist die Außenseite der ersten Vliesstofflage mit der Innenseite der Folienlage direkt und insbesondere flächenhaft verbunden.

Im Rahmen der vorliegenden Erfindung wird als "innen" dem Körper des Trägers der Inkontinenzwindel zugewandt verstanden. Als "außen" wird vom Körper des Trägers weggewandt verstanden.

In einer weiteren bevorzugten Ausführungsform ist die erste Vliesstofflage ausgewählt aus Vliesstoffen, welche mittels Wasserstrahlbindung (auch "Water-jet bonding", "Water entanglement" oder "Spunlace" genannt) gebunden wurden.

In einer weiteren bevorzugten Ausführungsform ist die erste Vliesstofflage ausgewählt aus einschichtigen Vliesstoffen.

In einer weiteren bevorzugten Ausführungsform ist die erste Vliesstofflage ausgewählt aus der Gruppe der einschichtigen Vliesstoffe, zu deren Herstellung ein Fasermaterial gewählt wurde, das aus einem einheitlichen Fasertyp oder einer Mischung verschiedener Fasertypen besteht.

In einer weiteren bevorzugten Ausführungsform weist die elastische Folienlage eine Dicke von 100 Mikrometern oder weniger, oder im Bereich zwischen 20 und 100 Mikrometern, oder im Bereich zwischen 20 und 70 Mikrometern, auf, wobei die Dicke mittels Mikroskop gemessen ist. Das Flächengewicht der elastischen Folienlage beträgt vorzugsweise 30-80 g/m$^2$, insbesondere 40-70 g/m$^2$.

In einer weiteren bevorzugten Ausführungsform ist das SIS-Triblock-Copolymer der elastischen Folienlage dadurch gekennzeichnet, dass es weniger als 10 Gew.-% oder weniger als 1 Gew.-% an Diblock-Bestandteilen beinhaltet.

In einer weiteren bevorzugten Ausführungsform umfasst oder besteht der Verbundwerkstoff aus zwei Vliesstofflagen und der elastischen Folienlage, nämlich der ersten Vliesstofflage und einer zweiten Vliesstofflage, die mit der dann zwischen den Vliesstofflagen angeordneten Folienlage direkt verbunden sind, wobei die erste Vliesstofflage unterhalb - mithin mit ihrer Außenseite auf der Innenseite (also der zum Träger zugewandten Seite) der elastischen Folienlage, und die zweite Vliesstofflage oberhalb, mithin mit ihrer Innenseite auf der Außenseite der elastischen Folienlage angeordnet und insbesondere jeweils flächenhaft verbunden ist.

Sämtliche vor- oder nachstehend beschriebene bevorzugten Ausführungsformen der Vliesstofflage(n) betreffen somit die erste und/oder gegebenenfalls die zweite Vliesstofflage.

Als Vliesstofflagen ("Non-woven fabrics") für die Anwendung innerhalb der Erfindung kommen grundsätzlich alle Vliesstoffe in Frage. Derartige Vliesstoffe sowie Verfahren zu ihrer Herstellung sind im Stand der Technik bekannt.

Als Fasermaterial für die Herstellung der Vliesstofflagen zur Verwendung in den erfindungsgemäßen Verbundwerkstoffen kommen grundsätzlich alle Fasermaterialien in Frage, die mittels der bekannten Herstellungsverfahren zu Vliesstoffen verarbeitet werden können. Ausgenommen hiervon sind lediglich mineralische Fasern und Glasfasern. Es können sowohl natürliche wie halbsynthetische oder vollsynthetische Fasern verwendet werden. Ebenso können einheitliche Fasermaterialien als auch Mischungen von verschiedenen Fasermaterialien eingesetzt werden. Geeignete Fasermaterialien sowie die Kriterien für ihren Einsatz sind dem Fachmann aus seinem allgemeinen Fachwissen geläufig.

Beispiele für geeignete Fasermaterialien sind vollsynthetische Fasern, wie etwa Polyethylen- , Polyethylenterephthalat-, Polypropylen-, Polyester- oder Polyamidfasern oder natürliche oder halbsynthetische Fasern wie Viskose-, Baumwoll-, Zellulose- oder Wollfasern. In einer besonders bevorzugten Ausführungsform bestehen die Fasern aus Polypropylen.

Auf dem Gebiet der Vliesherstellung sind zudem Zwei-Komponenten-Fasern bekannt, welche dem resultierenden Vlies besondere Eigenschaften verleihen können. Eine Untergruppe dieser Fasern sind die Zwei-Komponenten-Fasern aus zwei synthetischen Polymeren, etwa Polyester-Polyamid, Polyester-Polypropylen, Polyamid 6-Polyamid 6.6, und dergleichen. Ferner ist bekannt, dass derartige Zwei-Komponenten-Fasern bereits bei der Herstellung so modifiziert werden können, dass die zwei Komponenten in exakt definierten räumlichen Abschnitten der Fasern zu finden sind (z.B. als Kern und Hülle, als Segmente, und dergleichen). Alle derartigen Ausführungsformen sind grundsätzlich bei den Vliesstoffen für die Herstellung der erfindungsgemäßen Verbundwerkstoffe umfasst.

Vliesstoffe können zudem mittels bekannter Verfahren aus dem Stand der Technik sowohl einschichtig oder mehrschichtig hergestellt werden, d.h., es kann das Fasermaterial über den Querschnitt des Vliesstoffes unterschiedlich gewählt werden. Sowohl einschichtige als auch mehrschichtige Vliesstoffe sind für die erfindungsgemäßen Verbundwerkstoffe umfasst.

In einer bevorzugten Ausführungsform stellen die Vliesstoffe einschichtige Vliesstoffe dar, d.h., die Art und Zusammensetzung der Fasern ändern sich über den Querschnitt der Vliesstofflage(n) nicht.

Bei der Herstellung der Vliesstoffe können verschiedene Verfahren zum Einsatz kommen, um eine Bindung der Fasern untereinander bzw. mit dem Trägermaterial zu erreichen. Es kommen dabei mechanische, adhäsive oder thermische Verfahren zum Einsatz. Grundsätzlich sind für die Herstellung der Vliesstoffe für die Verwendung in den erfindungsgemäßen Verbundwerkstoffen alle dem Fachmann bekannten Verfahren wie beispielsweise Airlaying, Wetlaying, Carding, Spunbonding, Meltblowing, Hydroentangling, Vernadeln (Needling), Airthrough-Bonding oder Kombinationen davon geeignet.

In einer bevorzugten Ausführungsform der Erfindung sind die Vliesstoffe ausgewählt aus der Gruppe der mittels Wasserstrahlbindung ("Hydroentangling" oder auch "Water-jet bonding", "Water entanglement" oder "Spunlace" genannt) gebundenen Vliesstoffe.

Weiter bevorzugt sind die Vliesstoffe vor dem Hydroentangling durch Kardieren und/oder Luftlegeverfahren von Stapelfasern zu einer endlosen Faserlage vorgefertigt worden, welche dem Hydroentangling zugeführt wird. Hierbei erfolgt vorzugsweise eine Orientierung der Fasern in der Maschinen- also Längsrichtung der Vliesstoffherstellung. Bevorzugt sind die Fasern der Vliesstofflagen des Verbundwerkstoffes daher nicht wirr oder zufällig, sondern in der Längsrichtung der Vliesstoffherstellung orientiert.

Weiter vorzugsweise weisen die Vliesstoffe der Vliesstofflage(n) keine Muster, insbesondere kein mit bloßem Auge erkennbares Muster gebundener, insbesondere thermisch gebundener Bindestellen auf.

Insbesondere sind die Vliesstoffe der ersten oder zweiten Vliesstofflage nicht mittels thermischer Kalandrierung mit glatten und/oder gravierten Walzen verfestigt.

Besonders bevorzugt weisen die Vliesstoffe der ersten und/oder zweiten Vliesstofflage daher kein Punktbindemuster, mithin kein Muster thermisch oder durch Ultraschall gebundener Punkte (point-bonding) oder Fügebereiche beliebiger Geometrie (kreisförmig, rautenförmig, mehreckförmig, strichförmig, linienförmig oder Kombinationen davon) auf.

Besonders bevorzugt weisen die erste Vliesstofflage und/oder die zweite Vliesstofflage eine starke Anisotropie auf, derart, dass die Zugfestigkeit in der Querrichtung der Herstellung des Vliesstoffes geringer, insbesondere um mindestens den Faktor 1,5 oder den Faktor 2,0 oder den Faktor 3,0, oder den Faktor 3,5 geringer ist als die Zugfestigkeit in der Längsrichtung.

Bevorzugt beträgt die Zugfestigkeit (nach NWSP 110.4.R0.(15)) der ersten und ggf. der zweiten Vliesstofflage in der Querrichtung höchstens 20 N/5cm, oder höchstens 15 N/5cm oder höchstens 12 N/5cm.

Die Bruchdehnung (nach NWSP 110.4.R0.(15)) in der Querrichtung der Herstellung des Vliesstoffes beträgt vorzugsweise mindestens 130%, oder mindestens 150%. Vorteilhaft beträgt die Dehnung bei 5N in der Querrichtung mindestens 100% oder mindestens 120%.

Das Flächengewicht (nach NWSP 130.1.R.0.(15)) der ersten oder zweiten Vliesstofflage beträgt bevorzugt 10-50 g/m$^2$, oder 15-35 g/m$^2$ oder 18 -25 g/m$^2$.

Es ist vorteilhaft, die erste Vliesstofflage und/oder die zweite Vliesstofflage derart anzuordnen, dass die Längsrichtung der Herstellung der Vliesstofflage im Wesentlichen auch der Längsrichtung der Inkontinenzwegwerfwindel entspricht. Dies hat den Vorteil, dass die Vliesstofflagen einer Dehnung des Verbundwerkstoffes in der Querrichtung der Inkontinenzwegwerfwindel wenig Widerstand entgegensetzen.

Dessen ungeachtet ist es vorteilhaft, die elastische Folienlage derart anzuordnen, dass die Längsrichtung der Herstellung der elastischen Folienlage im Wesentlichen auch der Längsrichtung der Inkontinenzwegwerfwindel entspricht.

In Weiterbildung der Erfindung ist vorgesehen, dass der Verbundwerkstoff dadurch herstellbar insbesondere hergestellt ist, dass die elastische Folienlage durch Extrusionslaminierung auf die erste Vliesstofflage aufgebracht und mit der ersten Vliesstofflage insbesondere vollflächig verbunden wird, insbesondere zwischen die erste und die zweite Vliesstofflage eingebracht und dabei sowohl mit der ersten als auch mit der zweiten Vliesstofflage insbesondere vollflächig verbunden wird.

Bei der Extrusionslaminierung wird die Polymermischung als heißer, flüssiger Film auf bzw. zwischen die Vliesstofflage(n) insbesondere in einem Walzenspalt eingebracht, wobei sich beim Erkalten zwischen der Lage aus dem Polymer und der/den Vliesstofflage(n) unmittelbar eine direkte, insbesondere weitestgehend vollflächige Verbindung ergibt. Die Folienlage wird damit direkt, das heißt ohne einen weiteren Haftvermittler wie einen Klebstoff mit der/den Vlieslage(n) verbunden. Die Vliesstofflagen können nachgeordnet in einer Verbindungseinheit (z. Bsp. einem weiteren Walzenspalt), welche unmittelbar hinter der Extrusionseinheit angeordnet ist, mit dem noch wenig plastischen Film durch leichtes Aneinanderdrücken stärker verbunden werden. Alternativ ist es möglich, durch ein verstärktes Aneinanderpressen in ausgewählten Bereichen, die Vliesstofflagen stärker in den noch plastischen Film zu pressen als dieses in den übrigen Bereichen geschieht. Hierbei kann das Anpressen auch in einem separaten Schritt erfolgen, während der Film noch in einem plastischen Zustand ist.

Im Nachgang kann der Verbundwerkstoff optional partiell oder vollflächig mittels Vordehnung durch übliche Verfahren, wie Ring Rolling oder Dehnen mittels eines Spannrahmens oder ähnliches, aktiviert werden. Hierbei wird der Verbundwerkstoff, insbesondere in der Querrichtung der Herstellung des Verbundwerkstoffes erstmalig gedehnt, damit der Verbundwerkstoff bei bestimmungsgemäßer Verwendung als elastische Komponente einer Inkontinenzwegwerfwindel dem Dehnen in Gebrauch der Windel weniger Widerstand entgegensetzt.

Insbesondere erfordert die Extrusionslaminierung im Rahmen der vorliegenden Erfindung keine weitere Verbindung der den Verbundwerkstoff bildenden Lagen durch separate Klebe- oder Bindemittel oder sonstige Haftvermittler.

Zwar erweist es sich wie oben beschrieben als vorteilhaft, wenn der Lagenverbund unmittelbar nach dem Extrusionsvorgang einem Druck, insbesondere einem Flächen- oder Liniendruck ausgesetzt wird, insbesondere dadurch, dass der Verbund durch den Spalt zweier Walzen geführt wird; allerdings erfolgt vorzugsweise keine weitere Fügung der Lagen aneinander mittels thermischer Kalandrierung mit glatten und/oder gravierten Walzen oder vermittels Ultraschallschweißen. Besonders bevorzugt weist der Verbundwerkstoff daher kein Muster thermisch gebundener Punkte (point-bonding) beliebiger Geometrie (kreisförmig, rautenförmig, mehreckförmig, strichförmig, linienförmig oder Kombinationen davon) auf.

Wie oben erläutert ist die Inkontinenzwegwerfwindel als eine sogenannte tape-type Windel ausgeführt. Zum Anlegen und Schließen der Inkontinenzwegwerfwindel an einen Benutzer sind die hinteren Seitenabschnitte um den Körper des Benutzers herumlegbar und in überlappende Anordnung mit einer Außenseite des Vorderbereichs der Windel bringbar, an der sie dann über ein jeweiliges Verschlussmittel anheftbar sind.

Bevorzugt weisen die hinteren Seitenabschnitte daher insbesondere im Bereich ihres in Querrichtung freien Endes jeweils wenigstens ein Verschlussmittel auf.

Das Anfügen der hinteren Seitenabschnitte an den hinteren Längsrand des Hauptteils der Inkontinenzwegwerfwindel erfolgt insbesondere durch Thermoschweißen, Ultraschallschweißen oder Kleben.

Besonders bevorzugt ist die Tape-type-Windel mit Seitenabschnitten als sogenannte T-Form Windel ausgeführt. Solchenfalls sind an den Vorderbereich keine Seitenabschnitte angefügt, sondern die vorderen seitlichen Längsränder des Hauptteils bilden einen frei endenden Längsrand der Windel, wobei die hinteren Seitenabschnitte die elastische Komponente aufweisen und zum Anlegen und Schließen der Inkontinenzwegwerfwindel an einen Benutzer jeweils entlang einer Umfangsrichtung um den Körper des Benutzers herumlegbar und in überlappende Anordnung mit einer Außenseite des Vorderbereichs bringbar sind, an der sie dann über das jeweilige Verschlussmittel jeweils lösbar anhaftbar sind. Derartige T-Form Tape-type-Windel mit Seitenabschnitten sind beispielsweise in WO2017114695A1 offenbart.

Die hinteren Seitenabschnitte sind in der Längsrichtung der Windel zumeist ausladend, jedoch in der Regel kürzer als die Erstreckung der hinteren seitlichen Längsränder des Hauptteils im Rückenbereich. In der Querrichtung sind die hinteren Seitenabschnitte von T-Form-Windeln derart erstreckt, dass sie in Überlappung mit der Außenseite des Vorderbereichs des Hauptteils gebracht werden können, damit die im Bereich der jeweiligen freien Enden der hinteren Seitenabschnitte vorgesehenen Verschlussmittel auf die Außenseite des Hauptteils der Windel geschlossen werden können.

Im Unterschied hierzu sind sogenannte Gürtelwindeln in großem Umfang bekannt geworden, bei denen sich beidseits vom Rückenbereich des Windelhauptteils in Querrichtung sehr lange Gürtelabschnitte weg erstrecken, die derart bemessen sind, dass sie um den gesamten Bauchumfang des Benutzers herum auf sich selbst geschlossen werden können. Bei Anlegen einer Gürtelwindel wird das Produkt von hinten gegen den Hüft- oder Rückenbereich des Benutzers gelegt, und es werden sodann die beiden Gürtelabschnitte auf der Bauchseite des Benutzers direkt aufeinander geschlossen.

Nach einer weiteren bevorzugten Ausführungsform der Erfindung weist der Verbundwerkstoff nach der in der Beschreibung dargelegten Prüfmethode bei erstmaliger Dehnung in der Querrichtung um 140% eine Rückstellkraft von K140%,1 ≤ 10 N/25mm, insbesondere ≥ 2 N/25mm, weiter insbesondere ≥ 4 N/25mm auf.

Häufig werden die Inkontinenzwegwerfwindel beim Anlegen zumindest einmal nachjustiert. Es ist daher vorteilhaft, wenn der Verbundwerkstoff nach der in der Beschreibung dargelegten Prüfmethode bei zweiter Dehnung in der Querrichtung um 140% eine Rückstellkraft von K140%,2 ≤ 10 N/25mm, insbesondere ≤ 8 N/25mm, insbesondere ≥ 1,5 N/25mm, insbesondere ≥ 3,5 N/25mm aufweist.

Aus den vorgenannten Gründen ist es weiterhin vorteilhaft, wenn die elastische Komponente über einen großen Bereich der für die Praxis relevanten Dehnung der elastischen Komponente einen vergleichsweise flachen Kraftverlauf aufweist.

Es ist daher bevorzugt, dass der Verbundwerkstoff nach der in der Beschreibung dargelegten Prüfmethode einen ersten Kraftabfall DeltaK1 = K80%,1, 140% - K80%,2, 140% von ≤ 6 N/25mm, insbesondere von ≤ 5 N/25mm, insbesondere von ≤ 4 N/25mm, insbesondere von ≥ 0,5 N/25mm aufweist. (wobei K80%,1, 140% die Rückstellkraft bei erstmaliger Dehnung um 80% ist und K80%,2, 140% die Rückstellkraft bei zweiter Dehnung um 80% ist, bei maximaler Dehnung von 140% im oberen Umkehrpunkt des Dehnungszyklus nach der unten beschriebenen Prüfmethode).

Bevorzugt besitzt der Verbundwerkstoff nach der in der Beschreibung dargelegten Prüfmethode einen zweiten Kraftabfall DeltaK2 = K140%,2 - K20%,2, 140% von ≤ 9N N/25mm, insbesondere von ≤ 8N N/25mm, insbesondere von ≤ 7N N/25mm, insbesondere von ≥ 1,5N N/25mm, insbesondere von ≥ 2,5N N/25mm, insbesondere von ≥ 3,5N aufweist. (wobei K140%,2 die Rückstellkraft bei zweiter Dehnung um 140% ist und K20%,2, 140% die Rückstellkraft bei zweiter Dehnung um 20% ist, bei maximaler Dehnung von 140% im oberen Umkehrpunkt des Dehnungszyklus nach der unten beschriebenen Prüfmethode.).

Es hat sich als bevorzugt herausgestellt, wenn die elastische Komponente in Gebrauch bei Bedarf bis zu 200% dehnbar ist, ohne dass der Nutzer Gefahr läuft, das Material zu zerstören oder sehr starken Rückstellkräften ausgesetzt zu sein.

Daher ist vorteilhaft, wenn der Verbundwerkstoff nach der in der Beschreibung dargelegten Prüfmethode bei erstmaliger Dehnung in der Querrichtung um 200% eine Rückstellkraft von K200%,1 ≤ 14 N/25mm, insbesondere von K200%,1 ≤ 12 N/25mm, weiter insbesondere von K200%,1 ≤ 11 N/25mm, weiter insbesondere von K200%,1 ≥ 6 N/25mm, weiter insbesondere von K200%,1 ≥ 8 N/25mm aufweist.

Vorteilhaft ist des Weiteren, wenn der Verbundwerkstoff nach der in der Beschreibung dargelegten Prüfmethode bei zweiter Dehnung in der Querrichtung um 200% eine Rückstellkraft von K200%,2 ≤ 10 N/25mm, insbesondere von ≤ 8 N/25mm, insbesondere von ≥ 3,5 N/25mm, insbesondere von ≥ 5,5 N/25mm aufweist.

Nach einer bevorzugten Ausführungsform ist vorgesehen, dass der Verbundwerkstoff nach der in der Beschreibung dargelegten Prüfmethode einen dritten Kraftabfall DeltaK3 = K80%,1,200% - K80%,2, 200% von ≤ 6 N/25mm, insbesondere von ≤ 5 N/25mm, insbesondere von ≤ 4 N/25mm, insbesondere von ≥ 0,5 N/25mm aufweist. (wobei K80%,1 die Rückstellkraft bei erstmaliger Dehnung um 80% ist und K80%,2 die Rückstellkraft bei zweiter Dehnung um 80% ist, bei maximaler Dehnung von 200% im oberen Umkehrpunkt des Dehnungszyklus).

Weiter vorteilhaft ist, wenn der Verbundwerkstoff nach der in der Beschreibung dargelegten Prüfmethode einen vierten Kraftabfall DeltaK4 = K200%,2 - K20%,2,200% von ≤ 9 N/25mm, insbesondere von ≤ 8 N/25mm, insbesondere von ≤ 7 N/25mm, insbesondere von ≥ 1,5N N/25mm, insbesondere von ≥ 2,5 N/25mm, insbesondere von ≥ 3,5 N/25mm aufweist. (wobei K200%,2 die Rückstellkraft bei zweiter Dehnung um 200% ist und K20%,2, 200% die Rückstellkraft bei zweiter Dehnung um 20% ist, bei maximaler Dehnung von 200% im oberen Umkehrpunkt des Dehnungszyklus.).

Prüfmethode elastische Eigenschaften, Hysterese Test

Zur Ermittlung der elastischen Eigenschaften werden Hysterese Tests vorgenommen. Das zu prüfende Material wird unter definierten Bedingungen ein- oder mehrmals bis zu einem jeweils spezifizierten Maximalwert gedehnt und anschließend wieder entlastet. Es werden Rückstellkräfte bei definierten Dehnungswerten bei Belastung oder Entlastung gemessen. Für die Durchführung der Prüfungen wurde eine Zugprüfmaschine Typ Shimadzu AG-Xplus, gemäß DIN EN ISO 7500-01:2004-11, mit unterer fester Klemmbacke und oberer beweglicher Klemmbacke verwendet. Vorzugsweise verfügt die Testvorrichtung über eine Software, welche den Prüfzyklus steuert und ein automatisiertes Aufzeichnen der Kraftwerte sichergestellt.

Bei der Durchführung der Prüfmethode wird der zu prüfende rechteckige Materialabschnitt des elastischen Verbundwerkstoffs idealerweise mit einer Breite von 25 mm und einer Länge von 60 mm (die Länge entspricht der Zugrichtung) als Prüfling bereitgestellt. Hierzu wird der Prüfling zum Beispiel aus einer entsprechenden elastischen Komponente der Inkontinenzwegwerfwindel herausgestanzt, wobei die Zugrichtung des Prüflings der Querrichtung der Inkontinenzwegwerfwindel entspricht.

Es werden Hysteresezyklen wie folgt vorgenommen:

A Hysteresezyklus bis 140% Maximaldehnung:

1. Ein Prüfling des Verbundwerkstoffes mit einer Breite von 25 mm und einer Länge von ca. 60 mm wird in die Zugprüfmaschine fest eingespannt, wobei der Klemmbackenabstand 40 mm beträgt.
2. Vorspannung des Prüflings mit einer Kraft von 0,05 N (effektiver Klemmbackenabstand).
3. Dehnen (Belastung, 1. Zyklus) des Prüflings bis zur Maximaldehnung von 140%.
4. Es werden die Rückstellkräfte des 1. Zyklus bei Belastung bei 80% Dehnung K80%,1,140% und bei Maximaldehnung von 140% (d.h. im oberen Umkehrpunkt) K140%,1 aufgezeichnet.
5. Entlastung auf den effektiven Klemmbackenabstand bei Vorspannung (siehe 2.).
6. Dehnen (Belastung, 2. Zyklus) des Prüflings bis zu der Maximaldehnung von 140%.
7. Es werden die Rückstellkräfte des 2. Zyklus bei Belastung bei 20% Dehnung K20%,2,140% und bei 80% Dehnung K80%,2,140% und bei Maximaldehnung von 140% (d.h. im oberen Umkehrpunkt) K140%,2 aufgezeichnet.
8. Entlastung auf den effektiven Klemmbackenabstand bei Vorspannung (siehe 2.)
9. Dehnen (Belastung, 3. Zyklus) des Prüflings bis zu der Maximaldehnung von 140%.
10. Entlastung auf den effektiven Klemmbackenabstand bei Vorspannung (siehe 2.)

B Hysteresezyklus bis 200% Maximaldehnung:

1. Ein Prüfling des Verbundwerkstoffes mit einer Breite von 25 mm und einer Länge von ca. 60 mm wird in die Zugprüfmaschine fest eingespannt, wobei der Klemmbackenabstand 40 mm beträgt.
2. Vorspannung des Prüflings mit einer Kraft von 0,05 N (effektiver Klemmbackenabstand).
3. Dehnen (Belastung, 1. Zyklus) des Prüflings bis zur Maximaldehnung von 200%.
4. Es werden die Rückstellkräfte des 1. Zyklus bei Belastung bei 80% Dehnung K80%,1,200% und bei Maximaldehnung von 200 % (d.h. im oberen Umkehrpunkt) K200%,1 aufgezeichnet.
5. Entlastung auf den effektiven Klemmbackenabstand bei Vorspannung (siehe 2.)

6. Dehnen (Belastung, 2 Zyklus) des Prüflings bis zu der Maximaldehnung von 200%.

7. Es werden die Rückstellkräfte des 2. Zyklus bei Belastung bei 20% Dehnung K20%,2,200% und bei 80% Dehnung K80%,2,200% und bei Maximaldehnung von 200% (d.h. im oberen Umkehrpunkt) K200%,2 aufgezeichnet.

8. Entlastung auf den effektiven Klemmbackenabstand bei Vorspannung (siehe 2.)

9. Dehnen (Belastung, 3. Zyklus) des Prüflings bis zu der Maximaldehnung von 200%.

10. Entlastung auf den effektiven Klemmbackenabstand bei Vorspannung (siehe 2.)

Die Prüfgeschwindigkeit (Be- und Entlastung) der Hysteresezyklen A und B beträgt 500mm/min.

Load Cell (Kraftmessdose): Es ist eine geeignete Kraftmessdose für den jeweiligen Messbereich zu verwenden, kalibriert nach Genauigkeitsklasse 1 der DIN EN ISO 7500-1. Vorliegend wurde eine Kraftmessdose Typ Shimadzu SLBL-1KN verwendet.

Der zu prüfende Materialabschnitt des elastischen Verbundwerkstoffs wird über 24 h bei 23° C und 50 % relativer Luftfeuchte konditioniert. Die Prüfung findet ebenfalls bei 23° C und 50 % relativer Luftfeuchte statt.

Hier wie vor- und nachstehend wird die Dehnung in % nach folgender Formel berechnet:

(gedehnte Endlänge des Prüfabschnitts EL – Ausgangslänge des Prüfabschnitts unter Vorspannung AL) : Ausgangslänge des Prüfabschnitts unter Vorspannung AL) x 100 [%].

Beispiel:

Prüfabschnitt ungedehnt (das heißt nach Anlegen der Vorspannung), AL = 41 mm
Endlänge des Prüfabschnitts gedehnt EL = 98,4 mm
Dehnung = ((98,4-41):41) x 100[%] = 140%

Folgende Werte werden ermittelt:
A Hysteresezyklus bis 140% Maximaldehnung:

Kraftwerte K140%, 1
Kraftwerte K140%,2
Erster Kraftabfall DeltaK1 = K80%, 1,140% - K80%,2,140%
Zweiter Kraftabfall DeltaK2 = K140%,2 - K20%,2,140%

B Hysteresezyklus bis 200% Maximaldehnung:

Kraftwerte K200%,1
Kraftwerte K200%,2
Dritter Kraftabfall DeltaK3 = K80%,1,200% - K80%,2,200%
Vierter Kraftabfall DeltaK4 = K200%,2 - K20%,2,200%

Sollte kein 25 mm breiter Abschnitt der elastischen Komponente zur Verfügung stehen, wird ein entsprechend schmalerer Abschnitt verwendet. Solchenfalls werden die ermittelten Kraftwerte rechnerisch auf einen 25 mm breiten Abschnitt normiert, derart dass die gemessenen Kräfte mit dem Faktor f multipliziert werden, welcher sich ergibt aus f=25/x, wobei x die Breite des Prüflings gemessen in mm ist. Im Falle eines 12,5 mm breiten Prüflings beträgt der Faktor f mithin 2.

Sollte kein 60 mm langer Abschnitt der elastischen Komponente zur Verfügung stehen, wird ein entsprechend kürzerer Abschnitt verwendet, wobei der Klemmbackenabstand, mithin die effektive Länge des Prüfabschnitts ggf. auf bis zu 15mm zu verringern ist. Es ist sicherzustellen, dass der Abschnitt fest in die Klemmbacken eingespannt wird.

Zur Prüfung, ob ein Material als "elastisch" in der jeweiligen Richtung einzustufen ist, ist im Rahmen der vorliegenden Erfindung folgende Prüfung der bleibenden Dehnung (Permanent Set) vorzunehmen:
Idealerweise wird ein 25mm breiter und 60 mm langer (Zugrichtung) Prüfling wie zuvor beschrieben in die Klemmen der Zugprüfmaschine mit Klemmbackenabstand von 40mm eingespannt, eine Vorspannung von 0,05N angefahren und eine zyklische Bewegung (Be- und Entlastung, Prüfgeschwindigkeit 500mm/min), ohne Haltezeit im oberen Umkehrpunkt, zwischen L0 (Länge des Prüfabschnitts bei Vorspannung von 0,05N) und einer Maximaldehnung von 60% durchgeführt. Bei Entlastung wird die Endlänge L1 ebenfalls bei 0,05N notiert.

Als bleibende Dehnung (permanent set) PS wird berechnet:

$$PS = ((L1-L0):L0)\times100\ [\%].$$

Als elastisch in der jeweiligen Richtung wird ein Material dann angesehen, wenn die bleibende Dehnung weniger als 25% beträgt.

"Elastisch" und "elastisch dehnbar" wird im Rahmen der vorliegenden Erfindung synonym verwendet.

Sollte kein 25 mm breiter Abschnitt der zu prüfenden Komponente zur Verfügung stehen, wird ein entsprechend schmalerer Abschnitt verwendet. Sollte kein 60 mm langer Abschnitt der elastischen Komponente zur Verfügung stehen, wird ein entsprechend kürzerer Abschnitt verwendet, wobei der Klemmbackenabstand, mithin die effektive Länge des Prüfabschnitts ggf. auf bis zu 15 mm zu verringern ist. Es ist sicherzustellen, dass der Abschnitt fest in die Klemmbacken eingespannt wird.

Zur Prüfung, ob ein Material oder eine Komponente als "undehnbar" in der jeweiligen Richtung einzustufen ist, ist im Rahmen der vorliegenden Erfindung folgende Prüfung vorzunehmen:

Idealerweise wird ein 25mm breiter und 60 mm langer (Zugrichtung) Prüfling des Materials wie zuvor beschrieben unter Vorspannung von 0,05N in die Klemmen der Zugprüfmaschine mit Klemmbackenabstand von 40mm eingespannt und der Prüfling bis zu einer Kraft von 5N gedehnt (belastet, Prüfgeschwindigkeit 500mm/min). Sollte die Dehnung bei der Kraft von 5N weniger als 30% betragen, wird das Material im Rahmen der vorliegenden Erfindung als undehnbar bezeichnet. Im Falle, dass der Prüfling bei der Ausführung des vorstehenden Tests bricht, bevor die maximale Kraft von 5 N erreicht ist, wird das Material im Rahmen der vorliegenden Erfindung ebenfalls als "undehnbar" in der jeweiligen Richtung eingestuft.

Sollte kein 25 mm breiter Abschnitt der zu prüfenden Komponente zur Verfügung stehen, wird ein entsprechend schmalerer Abschnitt verwendet. Solchenfalls wird anstelle der Kraft von 5N der einzustellende Kraftwert errechnet, indem die Belastung von 5N um den Faktor f1 reduziert wird, welcher sich ergibt aus f1=25/x, wobei x die Breite des Prüflings gemessen in mm ist. Beispiel: ein 12,5 mm breiter Prüfling würde einer Kraft von 2,5N ausgesetzt werden.

Sollte kein 60 mm langer Abschnitt der elastischen Komponente zur Verfügung stehen, wird ein entsprechend kürzerer Abschnitt verwendet, wobei der Klemmbackenabstand, mithin die effektive Länge des Prüfabschnitts ggf. auf bis zu 15 mm zu verringern ist. Es ist sicherzustellen, dass der Abschnitt fest in die Klemmbacken eingespannt wird.

Nachfolgend werden weitere bevorzugte Ausführungsformen der Erfindung beschrieben für den Fall, dass die Inkontinenzwegwerfwindel als T-Form tape-type-Windel mit angefügten hinteren Seitenabschnitten ausgeführt ist, die Inkontinenzwegwerfwindel also keine vorderen Seitenabschnitte aufweist, wobei die elastische Komponente in den hinteren Seitenabschnitten angeordnet ist und dort elastische oder elastifizierte Bereiche bildet.

Solchen Falls weisen die hinteren Seitenabschnitte in eben ausgebreitetem jedoch nicht gedehnten Zustand eine Erstreckung Q in der Querrichtung über den jeweiligen hinteren seitlichen Längsrand eines Hauptteils hinaus auf, wobei eine Längsmittelachse LM der hinteren Seitenabschnitte die Erstreckung Q halbiert, mithin die Seitenabschnitte in einen inneren Halbabschnitt und einen äußeren Halbabschnitt teilt.

Die Erstreckung der hinteren Seitenabschnitte in der Querrichtung weist einen an den hinteren seitlichen Längsrand des Hauptteils anschließenden proximalen Abschnitt und einen an den proximalen Abschnitt anschließenden, frei endenden distalen Abschnitt auf. Hierbei gilt im Rahmen der vorliegenden Erfindung definitionsgemäß, dass der proximale Abschnitt sich in Querrichtung ausgehend von dem hinteren seitlichen Längsrand des Hauptteils über eine Länge erstreckt, die 65% der Erstreckung Q eines hinteren Seitenabschnitts beträgt. Das bedeutet, dass der distale Abschnitt sich über eine Länge erstreckt, welche 35 % der Erstreckung Q beträgt.

Es ist bevorzugt, dass die elastische Komponente der hinteren Seitenabschnitte vollständig innerhalb des proximalen Abschnitts, insbesondere vollständig innerhalb des inneren Halbabschnitt angeordnet ist und weiter insbesondere an den zugeordneten hinteren seitlichen Längsrand heranreicht oder einen Abstand von dem hinteren seitlichen Längsrand in der Querrichtung von höchstens 30 mm aufweist, und dass die jeweiligen hinteren Seitenabschnitte in dem gesamten äußeren Halbabschnitt, insbesondere in dem gesamten distalen Abschnitt in der Querrichtung der Inkontinenzwegwerfwindel im wesentlichen undehnbar, insbesondere unelastisch ausgebildet sind.

Bevorzugt umfassen die hinteren Seitenabschnitte in der Querrichtung mindestens zwei, insbesondere drei vormals separate Materialabschnitte, welche durch an sich bekannte Fügeverfahren, wie Kleben oder Thermoschweißen oder Ultraschallschweißen aneinandergefügt sind. Insbesondere weist ein jeweiliger Seitenabschnitt ausgehend von dem Hauptteil einen ersten in der Querrichtung unelastischen, insbesondere undehnbaren Materialabschnitt auf, mit dem der Seitenabschnitt an den Hauptteil angefügt ist. Daran schließt sich als die elastische Komponente in der Querrichtung ein mittlerer elastischer Materialabschnitt an, welcher durch den elastischen Verbundwerkstoff gebildet ist. Daran schließt sich in der Querrichtung ein zweiter unelastischer, insbesondere undehnbarer Materialabschnitt an, welcher insbesondere Träger des Verschlussmittels ist. Die Materialabschnitte sind vorzugsweise durch im Wesentlichen in der Längsrichtung verlaufende Fügenähte, insbesondere durch Thermoschweißen, Ultraschallschweißen oder Kleben aneinandergefügt.

Es wird weiter vorgeschlagen, die Seitenabschnitte so auszubilden, dass bei Einleitung üblicher die Gebrauchssituation simulierender Kräfte in der Querrichtung der jeweilige elastische oder elastifizierte Bereich in der Querrichtung um wenigstens 70 %, insbesondere um wenigstens 100 %, weiter insbesondere um wenigstens 140 %, weiter insbesondere um wenigstens 150%, weiter insbesondere um wenigstens 180% weiter insbesondere um wenigstens 200% dehnbar ist.

Nach einer bevorzugten Ausführungsform ist vorgesehen, dass der elastische oder elastifizierte Bereich der hinteren Seitenabschnitte die Längsmittelachse LM eines jeweiligen hinteren Seitenabschnittes überfängt, sich also in der Querrichtung über die Längsmittelachse LM hinaus in Richtung auf den freien Längsrand der hinteren Seitenabschnitte erstreckt. Vorzugsweise beträgt das Maß der Erstreckung über die Längsmittelachse LM hinaus höchsten 30 mm, insbesondere höchstens 20 mm, weiter insbesondere mindestens 5 mm.

Der Hauptteil der Inkontinenzwegwerfwindel umfasst vorzugsweise ein zumindest bereichsweise flüssigkeitsdurchlässiges Topsheet und ein zumindest bereichsweise flüssigkeitsundurchlässiges Backsheet, welche den Saugkörper sandwichartig umgeben. Hierzu erstrecken sich Topsheet und Backsheet zumindest in Querrichtung, vorzugsweise auch in Längsrichtung über die Konturränder des Saugkörpers hinaus, um einen entsprechenden Überhang zu bilden. In dem Überhang sind Topsheet und Backsheet vorzugsweise zumindest bereichsweise miteinander verbunden, insbesondere durch an sich bekannte Fügeverfahren wie Schweißen, Siegeln oder Kleben.

In vorteilhafter Weise weist der Hauptteil in dem Schrittbereich beidseitig an einen jeweiligen Längsrand des Schrittbereichs angrenzend je einen in Längsrichtung der Inkontinenzwegwerfwindel erstreckten elastischen oder elastifizierten Abschnitt, mithin einen elastischen oder elastifizierten Beinöffnungsabschnitt auf. "In Längsrichtung" bedeutet hierbei, dass der elastische oder elastifizierte Beinöffnungsabschnitt zumindest eine Komponente in Längsrichtung aufweist, mithin auch schräg oder gekrümmt zur Längsrichtung verlaufen kann.

Vorzugsweise sind hierzu dem Fachmann an sich bekannte elastische Fäden (Lycra® o.Ä.) in vorgespanntem Zustand an den Hauptteil bildenden Materialien fixiert, vorzugsweise an Top- und/oder Backsheet des Hauptteils, insbesondere in einem Bereich, in dem Top- und/oder Backsheet einen Überhang außerhalb der Konturränder des Saugkörpers bilden. Ein elastischer oder elastifizierter Beinöffnungsabschnitt kann nach einer Variante auch durch flachmaterial- oder bandförmige Materialien wie elastische Bänder, Folien, Vliesstoffe oder Schaumstoffe gebildet sein.

Der Saugkörper ist geeignet und dazu bestimmt Körperausscheidungen, insbesondere Körperflüssigkeiten, insbesondere Urin aufzunehmen und dauerhaft zu speichern. Der Saugkörper kann hierzu vorteilhaft superabsorbierendes Polymermaterial (SAP) enthalten, insbesondere zu 5-100 Gewichtsprozent, vorzugsweise zu 10-95 Gewichtsprozent, weiter vorzugsweise zu 15-90 Gewichtsprozent ganz besonders bevorzugt zu 20-80 Gewichtsprozent. Typischerweise kann das SAP-Material zumindest das 15-fache, insbesondere das 20-fache seines Gewichts an 0,9 gewichtsprozentiger Kochsalzlösung absorbieren (gemessen nach NWSP 242.0.R2(15%).

Das SAP-Material kann beispielsweise partikelförmig oder faserförmig oder blatt- oder schaumförmig ausgebildet sein.

Der Saugkörper kann weitere Materialien, wie Zellstofffasern (wood pulp) oder Kunststofffasern enthalten. Denkbar ist weiterhin, den Saugkörper durch Anordnung von ein oder mehreren Schichten verschiedenen Materials, insbesondere aus Vliesstoff auszubilden. Der Saugkörper ist vorzugsweise integraler Bestandteil des Hauptteils und solchen Falls herstellerseitig unlösbar mit den weiteren Komponenten des Hauptteils verbunden. Solchen Falls ist der Saugkörper vorzugsweise unlösbar mit einem Topsheet und/oder mit einem Backsheet des Hauptteils verbunden.

Nach einer alternativen Ausführungsform ist der Saugkörper von den weiteren Komponenten des Hauptteils lösbar, insbesondere zum Zwecke der getrennten Entsorgung des gebrauchten mit Körperflüssigkeiten beladenen Saugkörpers. In diesem Fall, kann es vorteilhaft sein, den Saugkörper herstellerseitig getrennt von den übrigen Komponenten des Hauptteils bereitzustellen und Mittel vorzusehen, den Saugkörper mit den übrigen Komponenten des Hauptteils erst verbraucherseitig zu verbinden. Die übrigen Komponenten des Hauptteils sind solchen Falls vorzugsweise bereits herstellerseitig und ein Windelchassis bildend vorkonfektioniert.

Die hinteren Seitenabschnitte sind im einfachsten und bevorzugten Fall rechteckförmig ausgebildet, d.h. sie werden durch in der Querrichtung und in der Längsrichtung der Inkontinenzwegwerfwindel verlaufende Ränder begrenzt. Vorzugsweise wird zumindest der proximale Abschnitt der Erstreckung Q der Seitenabschnitte von in der Querrichtung verlaufenden Rändern begrenzt.

Nach einer alternativen Ausführung können die Seitenabschnitte trapezförmig konturiert sein. Insbesondere kann vorgesehen sein, dass die Längserstreckung der Seitenabschnitte mit der Entfernung vom Hauptteil abnimmt.

Vorteilhaft ist ein jeweiliger elastischer oder elastifizierter Bereich der hinteren Seitenabschnitte rechteckförmig ausgebildet, das heißt die elastische Komponente ist durch in der Querrichtung und in der Längsrichtung der Inkontinenzwegwerfwindel verlaufende Ränder begrenzt. Nach einer alternativen Ausführung kann eine elastische Komponente trapezförmig konturiert sein. Insbesondere kann vorgesehen sein, dass die Längserstreckung des elastischen Bereichs mit der Entfernung vom Hauptteil abnimmt.

Weiter vorzugsweise erstreckt sich ein jeweiliger elastischer oder elastifizierter Bereich über die volle Länge der hinteren Seitenabschnitte (in der Längsrichtung der Inkontinenzwegwerfwindel).

Die Erstreckung eines jeweiligen elastischen oder elastifizierten Bereichs der hinteren Seitenabschnitte in der Querrichtung beträgt ungedehnt vorzugsweise 40 bis 120 mm, insbesondere 60 - 100 mm. Die maximale Erstreckung eines jeweiligen elastischen oder elastifizierten Bereichs der hinteren Seitenabschnitte in der Längsrichtung beträgt vorzugsweise 70 bis 250 mm , insbesondere 100 bis 200 mm, weiter insbesondere 120 bis 170 mm.

Die Erstreckung Q der eben ausgebreiteten jedoch nicht gedehnten hinteren Seitenabschnitte in der Querrichtung der Inkontinenzwegwerfwindel über den hinteren seitlichen Längsrand des Hauptteils hinaus beträgt vorzugsweise 130 bis 280 mm, insbesondere 170 bis 250 mm.

Die Erstreckung B der hinteren Seitenabschnitte in der Längsrichtung beträgt im Bereich der Anfügung an den Hauptteil vorzugsweise 70 bis 250 mm, insbesondere 100 bis 200 mm, weiter insbesondere 120 bis 170 mm.

Vorzugsweise ist eine Erstreckung QE eines jeweiligen elastischen oder elastifizierten Bereichs der hinteren Seitenabschnitte in der Querrichtung und eine maximale Erstreckung Q der hinteren Seitenabschnitte über den jeweiligen hinteren seitlichen Längsrand hinaus derart bemessen ist, dass das Verhältnis der Erstreckungen QE/Q zueinander $0,20 < QE/Q < 0,50$, insbesondere $0,30 < QE/Q < 0,45$ beträgt.

Es hat sich bei der erfindungsgemäßen T-förmigen Inkontinenzwegwerfwindel als besonders vorteilhaft erwiesen, wenn die Erstreckung Q der hinteren Seitenabschnitte in der Querrichtung über den jeweiligen hinteren seitlichen Längsrand hinaus und eine maximale Erstreckung B der hinteren Seitenabschnitte in der Längsrichtung derart bemessen ist, dass das Verhältnis der Erstreckungen Q/B zueinander $1,0 < Q/B < 2,0$ beträgt. Dieses Verhältnis ist bei Gürtelwindeln um ein Vielfaches größer.

Es wird weiter vorgeschlagen, dass die beiden hinteren Seitenabschnitte in der Längsrichtung einen Abstand zu einem hinteren Querrand des Hauptteils von wenigstens 1 mm, insbesondere von wenigstens 5 mm, insbesondere von wenigstens 10 mm, insbesondere von wenigstens 15 mm, insbesondere von höchstens 50 mm aufweisen. Hierdurch ist gewährleistet, dass die beim Anlegen über die Verschlussmittel ausgeübten und dabei in den Rückbereich des Hauptteils eingeleiteten Querzugkräfte auf einen größeren Abschnitt des Hauptteils "verteilt" werden.

Weiter erweist sich als vorteilhaft, wenn eine in der Querrichtung erstreckte und das jeweilige Verschlussmittel auf der schrittzugewandten Seite tangierende gerade Linie den Saugkörper schneidet. Dies kann vorzugsweise dann realisiert werden, wenn die beiden hinteren Seitenabschnitte wie vorstehend erwähnt einen Abstand in der Längsrichtung zum hinteren Querrand des Hauptteils aufweisen. Insbesondere ist vorgesehen, dass eine in der Querrichtung erstreckte und die Seitenabschnitte in der Längsrichtung, im Bereich der Anfügung an den Hauptteil halbierende gerade Linie den Saugkörper schneidet. Es stabilisiert die Anlage des Saugkörpers und unterstützt einen korrekten Sitz der Windel.

Es erweist sich weiter als vorteilhaft, wenn jeder hintere Seitenabschnitt genau ein Verschlussmittel aufweist. Es handelt sich bei den Verschlussmitteln typischerweise um eine Lasche aus einem ein- oder mehrschichtigen Flachmaterial, welches ausgehend von einer in der Regel um einen distalen Längsrand des betrachteten Seitenabschnitts nach innen auf den Seitenabschnitt eingefalteten Konfiguration in eine nach außen ausgefaltete Betriebsstellung entfaltbar ist. In an sich bekannter und daher nicht näher zu beschreibender Weise ist ein jeweiliges Verschlussmittel mit klebenden und/oder mechanisch haftenden Bereichen, Schichten oder Elementen ausgestattet, wie zum Beispiel Haken/Schlaufen-Materialien. Sofern der Seitenabschnitt genau ein Verschlussmittel aufweist, so erweist es sich als vorteilhaft, wenn dieses Verschlussmittel in der Längsrichtung ungefähr mittig in einem distalen Bereich des Seitenabschnitts vorgesehen ist. Weiter erweist es sich als vorteilhaft, wenn das betreffende Verschlussmittel eine Erstreckung in der Längsrichtung zwischen 25 % und 75 % der Erstreckung B des Seitenabschnitts in der Längsrichtung beträgt. Ferner sind die jeweiligen Verschlussmittel vorzugsweise im ein- und ausgefalteten Zustand rechteckförmig ausgebildet. Sie sind in der herstellerseitigen nicht aktiven Konfiguration vorzugsweise nach innen auf sich selbst eingefaltet.

Hinsichtlich der Abmessungen des Hauptteils der Inkontinenzwegwerfwindel hat es sich als vorteilhaft erwiesen, wenn die Erstreckung des Hauptteils in der Querrichtung im Rückenbereich und/oder im Vorderbereich 250 bis 550 mm, insbesondere 300 bis 520 mm beträgt. Vorzugsweise weisen der Vorderbereich und der Rückenbereiche des Hauptteils dieselbe Quererstreckung (gemessen in mm) auf.

Die Erstreckung des Hauptteils in der Längsrichtung beträgt vorzugsweise 700 bis 1200 mm, insbesondere 800 bis 1100 mm.

Der Hauptteil kann im Schrittbereich mit einer Verengung in Querrichtung, mithin mit einer Beinöffnungskontur versehen sein. In einer alternativen Ausführungsform ist der Hauptteil rechteckförmig ausgebildet.

Die elastischen oder elastifizierten Bereiche der hinteren Seitenabschnitte sind erfindungsgemäß durch die elastische Komponente, welche den elastischen Verbundwerkstoff nach einer oder mehrerer der oben beschriebenen Ausführungsformen umfasst oder daraus besteht, gebildet.

In der Gebrauchssituation werden die hinteren Seitenabschnitte in Überlappung mit der Außenseite des Vorderbereichs des Hauptteils gebracht, damit die im Bereich der jeweiligen freien Enden an beiden hinteren Seitenabschnitten vorgesehenen Verschlussmittel auf die Außenseite des Hauptteils der Windel geschlossen werden können. Hierzu sind die Verschlussmittel und mindestens ein Bereich der Außenseite des Hauptteils als Verschlusssystem ausgebildet. Insbesondere weisen die Verschlussmittel hierzu mechanische Verschlusselemente wie Kletthakenelemente auf, insbesondere auch in Kombination mit haftklebenden Bereichen, vermittels derer die Verschlussmittel lösbar haftend mit der Außenseite des Hauptteils in Eingriff bringbar sind. Hierzu hat es sich als vorteilhaft erwiesen, wenn die Außenseite des Hauptteils zumindest bereichsweise, vorzugsweise vollständig durch einen entsprechend ausgebildeten Vliesstoff gebildet ist. Alternativ ist möglich, ein separates Klettflauschelement auf der Außenseite des Hauptteils im Vorderbereich vorzusehen, welche als Landezone für die Verschlussmittel der Seitenabschnitte dient.

Obschon die hinteren Seitenabschnitte in der Querrichtung deutlich kürzer sind als bei Gürtelwindeln, erweist es sich als vorteilhaft, wenn die hinteren Seitenabschnitte herstellerseitig um wenigstens zwei in der Längsrichtung verlaufende Seitenabschnitt-Falzachsen auf sich selbst gefaltet sind und die so erhaltene gefaltete Konfiguration herstellerseitig vorzugsweise fixiert ist, etwa durch einzelne Fügestellen, insbesondere Klebe- oder Ultraschallschweißstellen, die gleichwohl zum Entfalten der Seitenabschnitte durch den Benutzer verhältnismäßig leicht, insbesondere in einem Zug, manuell lösbar sind. In diesem Fall erweist sich ein einziges in der Längsrichtung ungefähr mittig an den Seitenabschnitten positioniertes Verschlussmittel als vorteilhaft, wobei dann die Fügestellen das eingefaltete Verschlussmittel nicht erfassen, sondern in der Längsrichtung außerhalb des Verschlussmittels angeordnet sind. Wenn die gegeneinander anliegenden Teilbereiche um das wenigstens eine nach innen eingeschlagene Verschlussmittel herum oder in der Längsrichtung oberhalb oder unterhalb des eingeschlagenen Verschlussmittels durch die vorgenannten Maßnahmen lösbar fixiert sind, so bildet das eingeschlagene Verschlussmittel einen gut ergreifbaren Anfassbereich zum Entfalten des jeweiligen Seitenabschnitts.

Weiter erweist sich als vorteilhaft, wenn die hinteren Seitenabschnitte herstellerseitig um wenigstens zwei in der Längsrichtung verlaufende Seitenabschnitt-Falzachsen auf sich selbst gefaltet sind und durch diese Seitenabschnitt-Falzachsen aufeinander gefaltete Teilbereiche der hinteren Seitenabschnitte definiert und begrenzt werden, und dass ein in der Querrichtung außen liegender Teilbereich im wesentlichen undehnbar ausgebildet ist. Hierdurch werden insbesondere die Ergreifbarkeit und die Entfaltbarkeit sowohl des Seitenabschnitts als auch des eingefalteten Verschlussmittels verbessert.

Weiter erweist sich als vorteilhaft, wenn ein an den außenliegenden Teilbereich nach innen anschließender Teilbereich ausgehend von der äußeren in der Längsrichtung verlaufenden Falzachse mit wenigstens 40 % seiner Fläche undehnbar ausgebildet ist. Diese wenigstens 40 % der Fläche werden also ausgehend von der außenliegenden Falzachse bestimmt, indem man hiervon ausgehend eine gedachte parallele Linie zur der Faltachse quasi scannend in der Querrichtung nach innen bewegt bis sie auf einen dehnbaren Bereich stößt.

Es wird dann die gescannte Fläche ermittelt und ins Verhältnis zu der Gesamtaufsichtsfläche des Teilbereichs gesetzt. Hierdurch wird erreicht, dass der in der Querrichtung außen liegende Teilbereich und der daran nach innen anschließende Teilbereich über eine sehr große undehnbare Fläche (von wenigstens 40 % der Fläche des letztgenannten Teilbereichs), die demzufolge frei von elastischen oder elastifizierenden Elementen ist, flächenhaft aneinander anliegen.

Die Erstreckung U eines jeweiligen undehnbaren Bereichs des an den außenliegenden Teilbereich nach innen anschließenden Teilbereichs beträgt ausgehend von der äußeren in der Längsrichtung verlaufenden Falzachse in Querrichtung bis zum Beginn eines dehnbaren Bereichs vorzugsweise mindestens 15 mm, insbesondere mindestens 20 mm, weiter insbesondere mindestens 25 mm, weiter vorzugsweise mindestens 30 mm, vorzugsweise jedoch höchstens 100 mm, weiter vorzugsweise höchstens 70 mm.

Es wird weiter vorgeschlagen, dass eine jeweilige im ausgefalteten Zustand innenliegende, also dem hinteren seitlichen Längsrand des Hauptteils benachbarte Seitenabschnitt-Falzachse innerhalb des elastischen oder elastifizierten Bereichs des betreffenden Seitenabschnitts verläuft. Hierdurch kann die versteifende und an sich unerwünschte Wirkung einer jeden Faltung bei einem Flachmaterial reduziert werden.

Hingegen erweist es sich als vorteilhaft, wenn eine jeweilige im ausgefalteten Zustand in der Querrichtung weiter außen liegende Seitenabschnitt-Falzachse innerhalb des undehnbaren Bereichs der hinteren Seitenabschnitte verläuft. Dort kann nämlich die versteifende Wirkung der Falzachse durchaus erwünscht sein, da sich hierdurch die Einleitung der Zugkraft über die Verschlussmittel gleichmäßiger auf die Seitenabschnitte verteilt.

Es wird weiter vorgeschlagen, dass die hinteren Seitenabschnitte herstellerseitig um genau zwei in der Längsrichtung verlaufende Seitenabschnitt-Falzachsen auf sich selbst gefaltet sind, so dass genau drei Teilbereiche der Seitenabschnitte gebildet werden, und dass der mittlere Teilbereich ausgehend von der äußeren in der Längsrichtung verlaufenden Falzachse mit wenigstens 40 % seiner Fläche undehnbar ausgebildet ist.

Für die eingefaltete Konfiguration der hinteren Seitenabschnitte gilt vorzugsweise, dass eine Erstreckung A der auf sich selbst gefalteten hinteren Seitenabschnitte in der Querrichtung über den jeweiligen hinteren seitlichen Längsrand hinaus und eine Erstreckung B der auf sich selbst gefalteten hinteren Seitenabschnitte in der Längsrichtung derart bemessen ist, dass das Verhältnis der Erstreckungen A/B zueinander 0,5 < A/B < 1 beträgt.

Vor der herstellerseitigen Verpackung der Inkontinenzwegwerfwindeln wird vorzugsweise der Hauptteil herstellerseitig mitsamt den auf sich selbst gefalteten hinteren Seitenabschnitten um eine erste und eine zweite jeweils in der Längsrichtung verlaufende Hauptteil-Falzachse nach innen auf sich selbst gefaltet, derart dass die beidseitigen hinteren Seitenabschnitte in Dickenrichtung, also orthogonal zu einer die Längsrichtung und die Querrichtung einschließenden Ebene, in wenigstens teilweiser Überlappung zueinander zu liegen kommen. Weiter vorzugsweise wird vor der herstellerseitigen Verpackung der Inkontinenzwegwerfwindeln der Hauptteil herstellerseitig mitsamt den auf sich selbst gefalteten hinteren Seitenabschnitten und vorzugsweise im Anschluss an die zuvor beschriebene Faltung um in der Längsrichtung verlaufende Hauptteil-Falzachsen zusätzlich um eine oder zwei in der Querrichtung verlaufende Hauptteil-Falzachsen nach innen auf sich selbst gefaltet.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der erfindungsgemäßen Inkontinenzwegwerfwindel. In der Zeichnung zeigt:

Figur 1 eine Draufsicht auf eine erfindungsgemäße Inkontinenzwegwerfwindel im eben ausgebreiteten, jedoch nicht gedehnten Zustand;
Figur 2 eine Schnittansicht der Windel nach Figur 1 mit Schnittebene II-II;
Figur 3 eine schematische Darstellung der Windel im angelegten Zustand;
Figuren 4a, b, c jeweils eine vergrößerte und teilweise Darstellung der Windel nach Figur 1 im Bereich eines hinteren Seitenabschnitts in eben ausgebreitetem, jedoch nicht gedehnten Zustand mit Bemaßungen bzw. mit Falzachsen;
Figur 5 eine vergrößerte Darstellung der Windel nach Figur 1 im Bereich eines hinteren Seitenabschnitts in auf sich selbst gefalteter Konfiguration;
Figur 6 eine Schnittansicht mit Schnittebene VI-VI Figur 5;
Figur 7 eine Schnittansicht mit Schnittebene A-A der Figur 1;
Figur 8 Schematisch die Darstellung einer Vorrichtung zur Extrusionslaminierung;
Figur 9 eine Prüfvorrichtung;
Figuren 10a und 10b Darstellungen eines Prüflings für die Bestimmung der Glyceryl trioleat Resistenz;
Figur 11 eine erste Kraftkurve;
Figur 12 erste Hysteresekurven;
Figur 13 zweite Hysteresekurven.

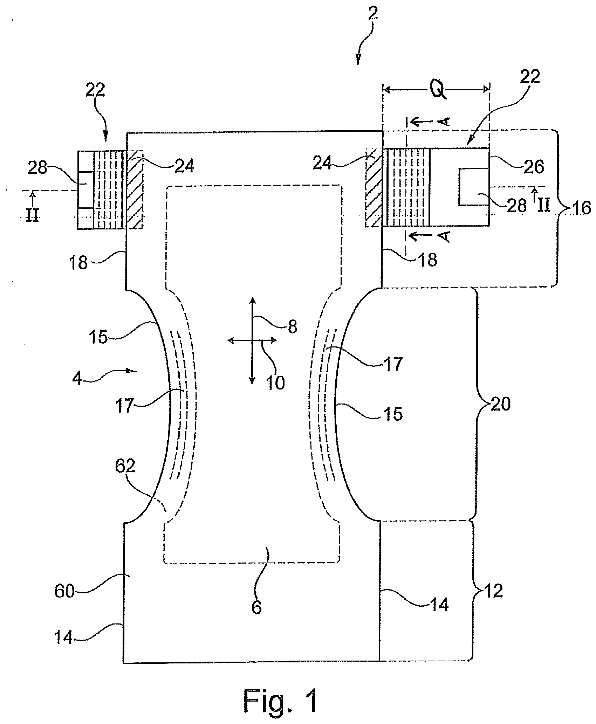

Die Figur 1 zeigt, nicht maßstäblich, sondern schematisch eine erfindungsgemäße insgesamt mit dem Bezugszeichen 2 bezeichnete tape-type Inkontinenzwegwerfwindel in der sogenannten T-Form. Die Windel 2 umfasst einen insgesamt mit dem Bezugszeichen 4 bezeichneten Hauptteil mit einem Körperflüssigkeiten absorbierenden Saugkörper 6. Der Saugkörper 6 umfasst vorzugsweise Zellulosefasern und superabsorbierende Polymerpartikel (SAP). Der Saugkörper 6 ist zwischen zwei Flachmaterialien, und zwar einer flüssigkeitsdurchlässigen Deckschicht 60 (Topsheet) und einer flüssigkeitsundurchlässigen Rückschicht 62 (Backsheet) des Windelhauptteils 4 angeordnet.

Bei der Windel 2 ist eine Längsrichtung 8 und eine Querrichtung 10 unterscheidbar, wobei letztere im angelegten Zustand der Windel der Hüftumfangsrichtung des Benutzers entspricht. Der Hauptteil 4 umfasst einen Vorderbereich 12 mit vorderen seitlichen Längsrändern 14, einen Rückenbereich 16 mit hinteren seitlichen Längsrändern 18 und einen dazwischen angeordneten Schrittbereich 20. An einem jeweiligen Längsrand 15 des Schrittbereichs 20 angrenzend weist der Hauptteil 4 je einen elastifizierten Abschnitt 17, mithin einen elastifizierten Beinöffnungsabschnitt auf. Diese elastifizierten Beinöffnungsabschnitte sind gebildet durch zwischen Topsheet 60 und Backsheet 62 verlaufende und in vorgespanntem Zustand an Topsheet 60 und Backsheet 62 fixierte elastische Fäden, die bogenförmig gekrümmt, mithin mit einer Komponente in Längsrichtung 8 orientiert sind.

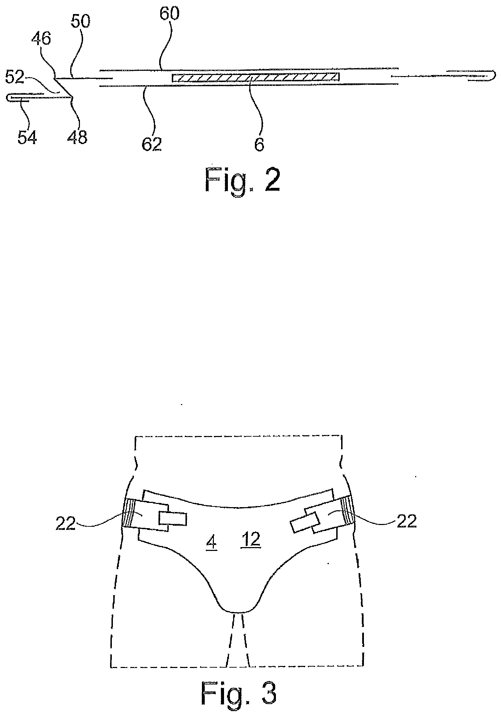

Bei der T-förmigen Windel 2 sind nur im Rückenbereich 16 des Hauptteils 4 in Querrichtung 10 seitlich über die hinteren seitlichen Längsränder 18 hinaus erstreckte hintere Seitenabschnitte 22 vorgesehen, die im Bereich der hinteren seitlichen Längsränder 18 in einem Überlappungsbereich 24 unlösbar an den Rückenbereich 16 des Hauptteils 4 angefügt sind. Die hinteren Seitenabschnitte 22 haben im Bereich ihres in Querrichtung 10 freien Endes 26 jeweils wenigstens ein Verschlussmittel 28. Das Verschlussmittel 28 ist in Form einer vorzugsweise rechteckigen Lasche ausgebildet und auf sich selbst eingefaltet. In der Gebrauchssituation lässt sich das Verschlussmittel öffnen, das heißt wieder auffalten, um die Inkontinenzwegwerfwindel 2 an einen Benutzer anzulegen, wobei die Seitenabschnitte 22 in Überlappung mit dem Vorderbereich 12 des Hauptteils 4 gebracht werden und die Verschlussmittel lösbar haftend auf der Außenseite des Vorderteils des Hauptteils festgelegt werden (schematisch dargestellt in Figur 3).

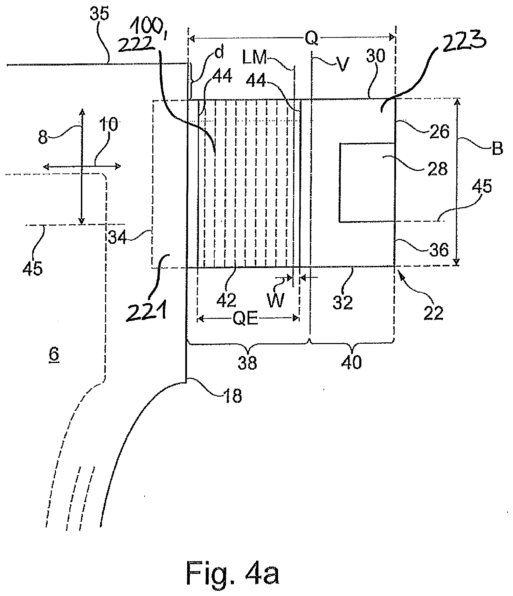

Die hinteren Seitenabschnitte 22 sind - wie am besten aus Figur 4a ersichtlich ist - vorzugsweise rechteckförmig ausgebildet, wobei sie durch in der Querrichtung 10 verlaufende Ränder 30, 32 und durch in der Längsrichtung 8 verlaufende Ränder 34, 36 begrenzt werden. In dem in Figur 4a dargestellten eben ausgefalteten oder ausgebreiteten jedoch nicht gedehnten Zustand haben die hinteren Seitenabschnitte 22 eine Erstreckung Q von 200 mm in der Querrichtung 10 über den hinteren seitlichen Längsrand 18 hinaus. Diese Erstreckung Q der hinteren Seitenabschnitte 22 in Querrichtung 10 außerhalb des Hauptteils 4 umfasst einen an den hinteren seitlichen Längsrand 18 anschließenden proximalen Abschnitt 38 und einen frei endende distalen Abschnitt 40 der Seitenabschnitte 22.

Der proximale Abschnitt 38 wird im Rahmen der vorliegenden Erfindung als derjenige Abschnitt definiert, welcher sich in Querrichtung 10 ausgehend von dem hinteren seitlichen Längsrand 18 des Hauptteils 4 über eine Länge erstreckt, die 65% der Erstreckung Q eines hinteren Seitenabschnitts 20 beträgt. Entsprechend ist der distale Abschnitt 40 derjenige Abschnitt, welcher sich in Querrichtung 10 an den proximalen Abschnitt anschließend bis zum freien Ende 26 des Seitenabschnitts 22 erstreckt, mithin über eine Länge von 35 % der Erstreckung Q. In Figur 4a markiert eine virtuelle in der Längsrichtung 8 verlaufende Linie V die Grenze zwischen dem proximalem Abschnitt (38) und dem distalem Abschnitt (40).

Die hinteren Seitenabschnitte 22 haben in der Längsrichtung 8 einen Abstand d von vorzugsweise 5-50 mm von einem hinteren Querrand 35 der Windel. Die Erstreckung B der hinteren Seitenabschnitte in der Längsrichtung beträgt im dargestellten Fall 140 mm.

Die hinteren Seitenabschnitte 22 sind in Querrichtung 10 außerhalb des Hauptteils 4 elastisch dehnbar ausgebildet. Sie umfassen hierfür einen elastischen oder elastifizierten Bereich 42. Dieser elastische oder elastifizierte Bereich 42 der hinteren Seitenabschnitte 22 ist vollständig innerhalb des proximalen Abschnitts 38 der hinteren Seitenabschnitte 22 angeordnet. Erweist im beispielhaft dargestellten Fall in der Querrichtung 10 einen geringen Abstand von wenigen Millimetern von dem hinteren seitlichen Längsrand 18 auf. Der elastische oder elastifizierte Bereich 42 ist rechteckförmig ausgebildet und seine Erstreckung QE in der Querrichtung ist durch in der Längsrichtung 8 erstreckte Ränder 44 begrenzt, wobei die Ränder 44 sich über die gesamte Längserstreckung der hinteren Seitenabschnitte 22 erstrecken.

In der dargestellten Ausführungsform der Inkontinenzwegwerf-windel erstreckt sich der elastische oder elastifizierte Bereich 42 um das Maß W = 10 mm über eine Längsmittelachse LM eines jeweiligen hinteren Seitenabschnittes 22 hinaus, überfängt also die Längsmittelachse LM, wobei die Längsmittelachse LM im Rahmen der vorliegenden Erfindung diejenige virtuelle, in der Längsrichtung 8 verlaufende Linie ist, welche einen hinteren Seitenabschnitt 22 in zwei in der Querrichtung 10 gleich lang erstreckte Hälften, nämlich den inneren Halbabschnitt und den äußeren Halbabschnitt teilt.

In dem distalen Abschnitt 40 der Erstreckung Q der hinteren Seitenabschnitte 22 sind die hinteren Seitenabschnitte 22 in der Querrichtung 10 undehnbar ausgebildet.

Der jeweilige elastische oder elastifizierte Bereich 42 der hinteren Seitenabschnitte 22 ist durch eine elastische Komponente gebildet, die aus einem elastischen Verbundwerkstoff 100 besteht. Auf den elastischen Verbundwerkstoff 100 wird unten noch näher eingegangen.

Die Seitenabschnitte 22 sind dann durch drei in der Querrichtung 10 hintereinander angeordnete und miteinander gefügte Materialabschnitte verschiedener Dehnbarkeit ausgebildet, wobei der mittlere Abschnitt im dargestellten Fall durch den elastischen Verbundwerkstoff 100 gebildet ist. So umfassen die hinteren Seitenabschnitte 22 in der dargestellten Ausführungsform einen ersten in der Querrichtung unelastischen, insbesondere undehnbaren Materialabschnitt 221. Daran schließt sich als die elastische Komponente in der Querrichtung ein mittlerer elastischer Materialabschnitt 222 an, welcher durch den elastischen Verbundwerkstoff 100 gebildet ist. Daran schließt sich in der Querrichtung ein zweiter unelastischer, insbesondere undehnbarer Materialabschnitt 223 an, welcher Träger des Verschlussmittels 28 ist. Die Materialabschnitte sind durch im Wesentlichen in der Längsrichtung verlaufende Fügenähte (nicht dargestellt) durch Thermoschweißen aneinandergefügt. Alternativ hierzu kann der elastische Verbundwerkstoff im vorgespannten Zustand mit Flachmaterialien der hinteren Seitenabschnitte 22 verbunden werden. Weiter alternativ ist es möglich, dass an sich undehnbare Flachmateriallaminate, wie beispielsweise Laminate umfassend undehnbare Vliesstoffe und der daran flächenhaft angefügte elastische Verbundwerkstoff der hinteren Seitenabschnitte 22 durch im Stand der Technik bekannte Maßnahmen, etwa durch das sogenannte "RingRolling", bereichsweise "aktiviert", das heißt elastisch dehnbar gemacht werden.

Ausführungsbeispiel AB eines elastischen Verbundwerkstoffes gemäß Erfindung:

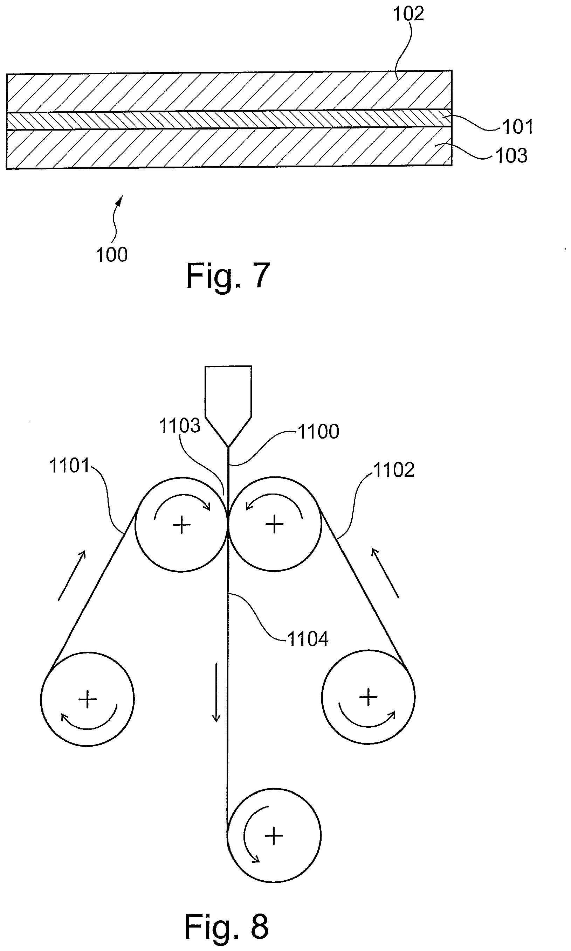

Figur 7 zeigt schematisch einen Querschnitt (entlang A-A der Figur 1) des elastischen Verbundwerkstoffes 100. Erkennbar sind eine elastische Folienlage 101 und eine erste Vliesstofflage 102 sowie eine zweite Vliesstofflage 103, welche mit je einer Oberseite (mit der Innenseite und der Außenseite) der elastischen Folienlage 101 durch Extrusionslaminierung verbunden sind. Die elastische Folie enthält keine medizinischen Weißöle. Die elastische Folie enthält außerdem keine technischen Weißöle (Tabelle 1).

Tabelle 1: Zusammensetzung der elastischen Folienlage eines elastischen Verbundwerkstoffes

| Komponenten der elastischen Folienlage von AB | Gew.-% |
|---|---|
| SIS | 30-40 |
| Poly(ethylen-co-propylen) | 50-70 |
| Additive (wie z.B. Farbpigmente, Stabilisatoren, Verarbeitungshilfen wie Wachse, Gleitmittel) | 0-10 |

Die Polymermischung der einschichtigen Folienlage enthält Styrol/Isopren/Styrol-Triblock-Copolymer (SIS) und ferner ein Poly(ethylen-co-propylen).

Die Dicke der elastischen Folienlage beträgt ca.67 μm. Das Flächengewicht der elastischen Folienlage beträgt 60g/m$^2$. Das Flächengewicht des Verbundwerkstoffes beträgt 110g/m$^2$.

Tabelle 2: Merkmale der ersten und zweiten Vliesstofflage des Verbundwerkstoffes VB

| | | Prüfmethode |
|---|---|---|
| **Vliesstoff-Typ** | Spunlace | |
| **Faserpolymer** | Polypropylen | |
| **Flächengewicht** | 25 g/m$^2$ | NWSP 130.1. R0(15) |
| **Bruchkraft (Tensile strength at Fmax), MD** | 35 N/5cm | NWSP 110.4. R0(15) |
| **Bruchkraft (Tensile strength at Fmax), CD** | 8,5 N/5cm | NWSP 110.4. R0(15) |
| **Bruchdehnung, MD** | 70% | NWSP 110.4. R0(15) |
| **Bruchdehnung, CD** | 180% | NWSP 110.4. R0(15) |
| **Dehnung bei 5 N/5cm, CD** | 145% | NWSP 110.4. R0(15) |

Die erste und die zweite Vliesstofflage 102, 103 sind identisch und als luftgelegte, einschichtige und nachfolgend wasservernadelte, sogenannte Spunlace-Nonwovens ausgeführt. Das Flächengewicht der Vliesstofflage beträgt jeweils 25 g/m$^2$. Weitere Parameter der Vliesstofflagen zeigt Tabelle 2.

Die erste und zweite Vliesstofflage 102, 103 weisen mithin eine starke Anisotropie auf, derart, dass die Zugfestigkeit in der Querrichtung der Herstellung des Vliesstoffes (CD) deutlich geringer ist als die Zugfestigkeit in der Längsrichtung (MD). Es ist vorteilhaft, die erste Vliesstofflage und ggf. die zweite Vliesstofflage derart anzuordnen, dass die Längsrichtung der Herstellung der Vliesstofflage (MD) der Längsrichtung der Herstellung des Verbundwerkstoffes und auch der Längsrichtung 8 der Inkontinenzwegwerfwindel entspricht. Dies hat den Vorteil, dass die Vliesstofflagen einer Dehnung des Verbundwerkstoffes in der Querrichtung des Verbundwerkstoffes und der Querrichtung der Inkontinenzwegwerfwindel wenig Widerstand entgegensetzen.

Die erste und zweite Vliesstofflage 102, 103 weisen kein Muster gebundener, insbesondere thermisch gebundener Bindestellen auf.

Der Verbundwerkstoff ist vorliegend durch Extrusionslaminierung, wie schematisch in Figur 8 dargestellt hergestellt.

Bei der an sich bekannten Extrusionslaminierung wird die Polymermischung als heißer, flüssiger Film 1100 auf bzw. zwischen die Vliesstofflagen 1101, 1102 in einem Walzenspalt 1103 eingebracht wird, wobei sich der Verbundwerkstoff 1104 beim Erkalten zwischen der Lage aus dem Polymer und den Vliesstofflagen unmittelbar durch eine direkte, insbesondere weitestgehend vollflächige Verbindung ergibt.

Die Vliesstofflagen können falls erforderlich nachgeordnet in einer Verbindungseinheit (z. Bsp. einem weiteren Walzenspalt, nicht dargestellt), welche unmittelbar hinter der Extrusionseinheit angeordnet ist, mit dem noch plastischen Film durch leichtes Aneinanderdrücken noch intensiver verbunden werden.

Im Nachgang kann der Verbundwerkstoff optional partiell oder vollflächig mittels Vordehnung quer zur Maschinenrichtung (der Herstellung des Verbundwerkstoffes) durch an sich übliche Verfahren, wie Ring Rolling oder Dehnen mittels eines Spannrahmens oder ähnliches, aktiviert werden. Hierbei wird zum einen der Zusammenhalt der Fasern innerhalb jeder der Vliesstofflagen in der Querrichtung zumindest partiell aufgebrochen, so dass dieVliesstofflagen der gewünschten Dehnung des Verbundwerkstoffes wenig Kraft entgegensetzen. Zum anderen wird durch die Aktivierung auch die elastische Folienlage erstmalig gedehnt, so dass sie jeder weiteren Dehnung (dann in Gebrauch) weniger Widerstand entgegensetzt (Luftballon-Effekt).

Insbesondere erfordert die Extrusionslaminierung im Rahmen der vorliegenden Erfindung keine weitere Verbindung der den Verbundwerkstoff bildenden Lagen durch separate Klebe- oder Bindemittel oder sonstige Haftvermittler oder durch musterartiges "point-bonding" durch Thermo- oder Ultraschallschweißen.

Der elastische Verbundwerkstoff des elastischen Bereichs der Seitenabschnitte wurde den weiter oben beschriebenden Tests unterzogen:

1. Glyceryl trioleat Resistenz

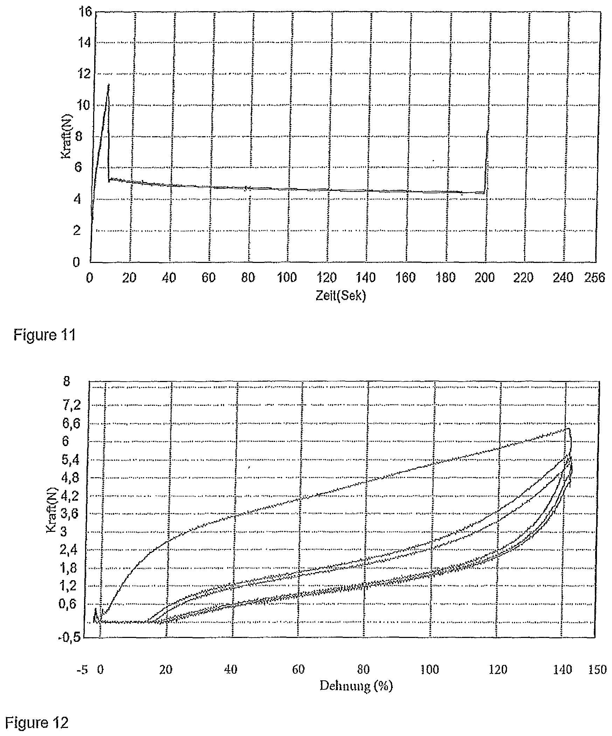

Figur 11 zeigt den Kraftverlauf, also die Rückstellkraft, die der Verbundwerkstoff der Belastung (Dehnung) unter dem Einwirken von Glyceryl trioleat entgegenzusetzen vermag.

Rückstellkraft K150%: 11,2 N/40mm
Rückstellkraft KGly150%: 8,0 N/40mm
Rückstellkraft KGly140%: 4,4 N/40mm
Glyceryl trioleat Resistenz RGly150% = (KGly150%) / K150%) $\times$ 100 [%] = 71,4 %

2. Elastische Eigenschaften - Hysterese

A Hysterese bis 140% Maximaldehnung

Figur 12 zeigt die Hysteresekurve des Verbundvliesstoffes, 1., 2. und 3. Zyklus., Maximaldehnung (oberer Umkehrpunkt) bei 140% Dehnung
Ergebnisse Hysteresezyklus bis 140% Maximaldehnung:

Rückstellkraft K140%,1: 6,41 N/25mm
Rückstellkraft K140%,2: 5,47 N/25mm
Erster Kraftabfall DeltaK1 = K80%,1,140% - K80%,2,140% = 2,58 N/25mm
Zweiter Kraftabfall DeltaK2 = K140%,2 - K20%,2,140% = 4,95 N/25mm

B Hysterese bis 200% Maximaldehnung

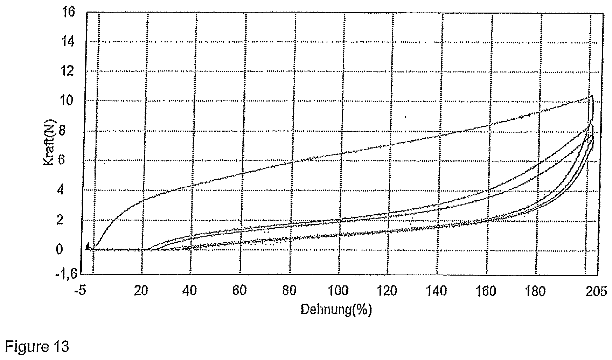

Figur 13 zeigt die Hysteresekurve des Verbundvliesstoffes, 1., 2. und 3. Zyklus., Maximaldehnung (oberer Umkehrpunkt) bei 200% Dehnung
Ergebnisse Hysteresezyklus bis 200% Maximaldehnung:

Rückstellkraft K200%,1: 10,31 N/25mm
Rückstellkraft K200%,2: 8,32 N/25mm
Dritter Kraftabfall DeltaK3 = K80%,1,200% - K80%,2,200% = 4,10 N/25mm
Vierter Kraftabfall DeltaK4 = K200%,2 - K20%,2,200% = 8,31 N/25mm

Der elastische Verbundwerkstoff wurde außerdem hinsichtlich seiner Beständigkeit gegenüber Mandelöl und Paraffinöl untersucht nach dem oben beschriebenen Test.
Die Ergebnisse zeigt Tabelle 3.

Tabelle 3

| Testöl | Verbundwerkstoff AB |
|---|---|
| Mandelöl | Keine Lochbildung innerhalb 3 h |
| Paraffinöl | Keine Lochbildung innerhalb 3 h |

Die gleichen Testergebnisse (keine Lochbildung innerhalb 3h) wurden auch mit verschiedenen kommerziellen Kosmetikprodukten als Testflüssigkeiten erhalten, die zusätzlich zu den genannten Testölen noch folgende Basisöle (teilweise in Form von Ölmischungen) enthielten: Rapsöl, Palmkern- oder Kokosöl, Avocadoöl, Sonnenblumenöl, Olivenöl, Sheabutter, und/oder Ringelblumenöl.

Vergleichsbeispiele

VB1: Als Vergleichsbeispiel VB1 wurde der die elastische Komponente der Seitenabschnitte bildende elastische Verbundwerkstoff einer T-Form tape-type Inkontinenzwindel, namentlich der Molicare® Premium Elastic, Größe L der PAUL HARTMANN AG, Deutschland, hinsichtlich der Resistenz gegenüber Glyzeryl trioleat untersucht. Ergebnisse waren wie folgt:

Rückstellkraft K150%: 13,0 N/40mm
Rückstellkraft KGly150%: 1,0 N N/40mm
Rückstellkraft KGly140%: 0,2 N/40mm
Glyceryl trioleat Resistenz RGly150% = (KGly150%) / K150%) $\times$ 100 [%] = 7,7 %.

Der Verbundwerkstoff des Vergleichsbeispiels VB1 umfasste einen Lagenverbund bestehend aus einer elastischen Folienmittellage aus einem Styrol-Blockcopolymer (SBC) einer Dicke von 105 μm, welche sandwichartig zwischen zwei Spinnvliesstofflagen angeordnet ist. Die Vliesstofflagen bestehen aus Polypropylenfilamenten mit einem Flächengewicht von jeweils 17 g/m². Der Lagenverbund ist durch ring rolling aktiviert.

VB2: Als Vergleichsbeispiel VB2 wurde ein elastischer Verbundwerkstoff erhalten von der Firma Lohmannkoester GmbH & Co. KG, Altendorf, Deutschland, untersucht. Die Zusammensetzung und Herstellung des Verbundwerkstoffes des VB2 war identisch zu Verbundwerkstoff des Ausführungsbeispiels AB, abgesehen von der Zusammensetzung der elastischen Folienlage wie in Tabelle 4 dargestellt:

Tabelle 4

| Komponenten der elastischen Folienmittellage von VB2 | Gew.-% |
|---|---|
| Styrol-Block-Copolymere | 40-60 |
| Medizinische Weißöle | 20-40 |
| Poly(ethylen-co-propylen) | 10-20 |
| Additive (wie z.B. Farbpigmente, Stabilisatoren, Verarbeitungshilfen wie Wachse, Gleitmittel) | 0-10 |

Die elastische Folienlage von VB2 enthält signifikante Anteile an medizinischen Weißölen als Weichmacher.

Der Verbundwerkstoff VB2 wurde auch hinsichtlich seiner Beständigkeit gegenüber Mandelöl und Paraffinöl untersucht nach dem oben beschriebenen Prüfverfahren. Die Ergebnisse zeigt Tabelle 5.

Tabelle 5

| Testöl | Verbundwerkstoff VB2 |
|---|---|
| Mandelöl | Bildung von Löchern innerhalb < 3h |
| Paraffinöl | Bildung von Löchern innerhalb < 3h |

Dieselben Testergebnisse (Bildung von Löchern) wurden auch mit verschiedenen kommerziellen Kosmetikprodukten als Testflüssigkeiten erhalten, die zusätzlich zu den genannten Testölen noch folgende Basisöle (teilweise in Form von Ölmischungen) enthielten: Rapsöl, Palmkern- oder Kokosöl, Avocadoöl, Sonnenblumenöl, Olivenöl, Sheabutter, und/oder Ringelblumenöl.

Weiter wurden die Verbundwerkstoffe AB (= Ausführungsbeispiel gemäß Erfindung) und Vergleichsbeispiel VB1 einem Testverfahren unterzogen, das dem oben beschriebenen Testverfahren zur Glyzeryl triolat Resistenz entsprach, wobei anstelle des Glyzerin triolat handelsübliche Pflegemittel (PM) als Testsubstanz verwendet wurden und wobei die Resistenz gegenüber der Testsubstanz nicht bei Dehnung von 150%, sondern bei 140% wie folgt ermittelt wurde:

- Dehnen um 140%, Kraftwert bei 140% Dehnung auslesen (= K 140%)
- Aufgabe von 200 μl des jeweiligen Pflegemittels PM
- 180 Sekunden Haltezeit bei 140%, dann Kraftwert bei 140% Dehnung auslesen (= K PM 140%)
- Berechnen von R PM 140% = (K PM 140% / K 140%) * 100 [%]

Tabelle 6 zeigt die ermittelten Resistenzen R PM 140%.

Tabelle 6 Resistenzen gegenüber handelsüblichen Pflegemitteln

| | K140% [N/40mm] | R PM 140% [%] | | | | | |
|---|---|---|---|---|---|---|---|
| Pflegemittel PM | | Menalind Professional Protect Öl-Hautschutzsp ray | Seni Care Regenrati on Skin Care Oil | Senslind Bodycare Hautpflege eöl | addermis biAktiv Aceite protectr hidrante pulverizad or | Hydro Vital Premium Hatpflege öl | |
| AB | 4,8 | 68,1 | 67,5 | 72,0 | 69,5 | 69,8 | |
| VB1 | 8,2 | 1,6 | 1,9 | 2,5 | 2,2 | 1,5 | |

Die Ergebnisse zeigen darüber hinaus, dass die Glyceryl triolat Resistenz nach der oben beschriebenen Prüfmethode ein guter Indikator für die Beständigkeit gegenüber handelsüblichen Pflegemitteln ist.

Die hinteren Seitenabschnitte 22 sind weiter derart am Rückenbereich 16 des Hauptteils 4 angeordnet, dass eine in der Querrichtung 10 erstreckte und das jeweilige Verschlussmittel 28 auf der schrittzugewandten Seite tangierende gerade Linie 45 den Saugkörper 6 schneidet.

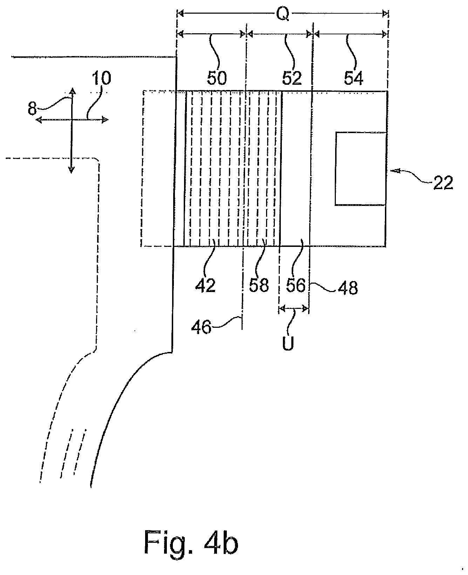

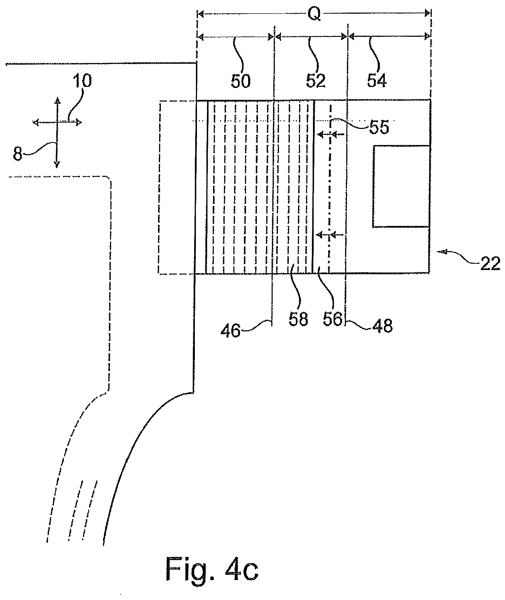

Wie in Figuren 1, 2 (jeweils links) und Figuren 4b, 5 und 6 dargestellt, sind die hinteren Seitenabschnitte 22 herstellerseitig um wenigstens zwei in der Längsrichtung 8 verlaufende Seitenabschnitt-Falzachsen 46, 48 auf sich selbst gefaltet. Die Seitenabschnitt-Falzachsen 46, 48 definieren und begrenzen dabei aufeinander gefaltete Teilbereiche 50, 52, 54 der hinteren Seitenabschnitte 22 (Figur 4b). Man erkennt, dass die dem hinteren seitlichen Längsrand 18 benachbarte, also innenliegende Seitenabschnitt-Falzachse 46 innerhalb des elastischen oder elastifizierten Bereichs 42 verläuft, während die in der Querrichtung 10 weiter außen liegende Seitenabschnitt-Falzachse 48 außerhalb des elastischen oder elastifizierten Bereichs 42, also innerhalb eines in der Querrichtung 10 undehnbaren Bereichs der Seitenabschnitte 22 verläuft. Der Teilbereich 52, welcher sich an den in der Querrichtung 10 außenliegenden Teilbereich 54 nach innen anschließt, ist ausgehend von der äußeren in der Längsrichtung 8 verlaufenden Falzachse 48 mit wenigstens 40 % seiner Fläche in Querrichtung 10 undehnbar ausgebildet. Zur Bestimmung dieser undehnbaren Fläche wird von der äußeren Falzachse 48 ausgehend und parallel hierzu eine gedachte Linie 55 quasi scannend in der Querrichtung 10 in Richtung auf den Hauptteil 4 nach innen bewegt (dies ist in Figur 4c durch Pfeile veranschaulicht) bis sie auf einen dehnbaren Bereich 58 stößt. Die so gescannte Fläche beträgt wenigstens 40 % der gesamten Aufsichtsfläche des Teilbereichs 52 im nicht gedehnten Zustand. Der Teilbereich 52 weist hier einen rechteckförmigen in der Querrichtung 10 äußeren undehnbaren Bereich 56 einer Quererstreckung U und einen rechteckförmigen in der Querrichtung 10 inneren elastisch dehnbaren Bereich 58 auf, die durch eine in der Längsrichtung 8 verlaufende gedachte Linie voneinander abgegrenzt sind. Dabei umfasst der rechteckförmige äußere undehnbare Bereich 56 - wie ausgeführt - wenigstens 40 % gesamten Aufsichtsfläche des Teilbereichs 52 im nicht gedehnten Zustand. Es ist auch denkbar, dass der sich an den außen liegenden Teilbereich 54 nach innen anschließende Teilbereich 52 über seine gesamte Erstreckung in Querrichtung 10 undehnbar ausgebildet ist.

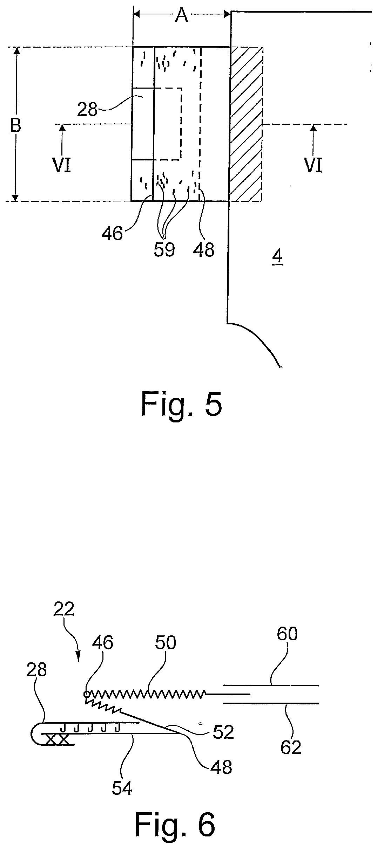

Die Figuren 5 und 6 zeigen schematisch die auf sich selbst gefaltete Konfiguration der hinteren Seitenabschnitte 22. Die gegeneinander gefalteten Teilbereiche 50, 52, 54 sind an in Figur 5 dargestellten Fügestellen 59 lösbar aneinander fixiert. Diese Fügestellen 59 sind durch eingangs beschriebene Maßnahmen ausgebildet. Es erweist sich als vorteilhaft, dass im Bereich der Überlappung der Teilbereiche 52, 54 zu einem wesentlichen Anteil undehnbare Bereiche miteinander lösbar gefügt sind. Man erkennt in Figur 6 auch in schematischer Darstellung die Anfügung des Verschlussmittels 28 beispielhaft an die körperabgewandte Außenseite des Seitenabschnitts 22 und die Umfaltung auf die körperzugewandte Seite des Seitenabschnitts 22. Ferner erkennt man die schematisch dargestellte Anfügung der hinteren Seitenabschnitte 22 beispielhaft zwischen zwei Flachmaterialien, beispielsweise eine flüssigkeitsdurchlässige Deckschicht 60 (Topsheet) und eine flüssigkeitsundurchlässige Rückschicht 62 (Backsheet) des Windelhauptteils 4.

Die Abmessung A der gefalteten Konfiguration der hinteren Seitenabschnitte 22 in der Querrichtung 10 außerhalb des Hauptteils 4 und die Abmessung B in der Längsrichtung 8 sind in Figur 5 dargestellt. Das Verhältnis A/B beträgt vorzugsweise 0,5 < A/B < 1. Das entsprechende Verhältnis Q/B der Seitenabschnitte 22 im eben ausgebreiteten jedoch nicht gedehnten Zustand beträgt vorzugsweise 1,0 < Q/B < 2,0 und ist in Figur 4a dargestellt. Das Verhältnis von QE, also der Erstreckung des elastischen oder elastifizierten Bereichs 42 in der Querrichtung, zu Q beträgt vorzugsweise 0,20 < QE/Q < 0,50, insbesondere 0,30 < QE/Q < 0,45.

## Patentansprüche

1. Inkontinenzwegwerfwindel (2) mit einer Längsrichtung (8) und einer Querrichtung (10) und mit einem einen Saugkörper (6) aufweisenden Hauptteil (4) umfassend einen Vorderbereich (12) mit vorderen seitlichen Längsrändern und einen Rückenbereich (16) mit hinteren seitlichen Längsrändern (18) und einen dazwischen angeordneten, zwischen den Beinen eines Benutzers zu liegen kommenden Schrittbereich und mit beidseits an den Rückenbereich (16) angefügten hinteren Seitenabschnitten (22), welche sich in der Querrichtung (10) über die seitlichen hinteren Längsränder (18) des Hauptteils (4) hinauserstrecken, und wobei die hinteren Seitenabschnitte (22) eine in der Querrichtung (10) elastische Komponente aufweisen, wobei die elastische Komponente einen elastischen Verbundwerkstoff (100) umfasst, wobei der Verbundwerkstoff (100) eine elastische Folienlage (101) und eine mit der elastischen Folienlage (101) verbundene erste Vliesstofflage (102) umfasst, wobei die Folienlage (101) einen einschichtigen Aufbau aufweist, wobei die erste Vliesstofflage (102) direkt mit der elastischen Folienlage (101) verbunden ist, wobei der Verbundwerkstoff (100) bei erstmaliger Dehnung in der Querrichtung (10) um 140% eine Rückstellkraft von K140%,1 ≤ 12 N/25mm aufweist und wobei der Verbundwerkstoff (100) eine Glyceryl trioleat Resistenz RGly150% von ≥ 50% aufweist und wobei K140%,1 und RGly150% nach der in der Beschreibung jeweils dargelegten Prüfmethode zu ermitteln sind.

2. Inkontinenzwegwerfwindel (2) nach Anspruch 1, wobei der Verbundwerkstoff (100) nach der in der Beschreibung dargelegten Prüfmethode bei erstmaliger Dehnung in der Querrichtung (10) um 140% eine Rückstellkraft von K140%,1 ≤ 10 N/25mm, insbesondere ≥ 2 N/25mm, weiter insbesondere ≥ 4 N/25mm aufweist, und weiter insbesondere nach der in der Beschreibung dargelegten Prüfmethode bei zweiter Dehnung in der Querrichtung (10) um 140% eine Rückstellkraft von K140%,2 ≤ 10 N/25mm, insbesondere ≤ 8 N/25mm, weiter insbesondere ≥ 1,5 N/25mm, weiter insbesondere ≥ 3,5 N/25mm aufweist.

3. Inkontinenzwegwerfwindel (2) nach einem oder mehreren der vorgenannten Ansprüche, wobei der Verbundwerkstoff (100) nach der in der Beschreibung dargelegten Prüfmethode einen ersten Kraftabfall DeltaK1 = K80%,1, 140% - K80%,2, 140% von ≤ 6 N/25mm, insbesondere von ≤ 5 N/25mm, insbesondere von ≤ 4 N/25mm, insbesondere von ≥ 0,5 N/25mm aufweist und weiter insbesondere nach der in der Beschreibung dargelegten Prüfmethode einen zweiten Kraftabfall DeltaK2 = K140%,2 - K20%,2, 140% von ≤ 9 N/25mm, insbesondere von ≤ 8 N/25mm, insbesondere von ≤ 7 N/25mm, insbesondere von ≥ 1,5 N/25mm, insbesondere von ≥ 2,5 N/25mm, insbesondere von ≥ 3,5N aufweist.

4. Inkontinenzwegwerfwindel (2) nach einem oder mehreren der vorgenannten Ansprüche, wobei der Verbundwerkstoff (100) nach der in der Beschreibung dargelegten Prüfmethode eine Glyceryl trioleat Resistenz RGly150% von ≥ 55%, weiter insbesondere von ≥ 60%, weiter insbesondere von ≥ 65%, weiter insbesondere von ≤ 95%, weiter insbesondere von ≤ 90%, weiter insbesondere von ≤ 85% aufweist.

5. Inkontinenzwegwerfwindel (2) nach einem oder mehreren der vorgenannten Ansprüche, wobei der Verbundwerkstoff (100) nach der in der Beschreibung dargelegten Prüfmethode eine Rückstellkraft KGly150% von 2,5 - 12,5 N/40mm, insbesondere von 5,5 - 10,5 N/40mm aufweist und weiter insbesondere nach der in der Beschreibung dargelegten Prüfmethode eine Rückstellkraft KGly140% von 1,5 - 7,0 N/40mm, insbesondere von 3,0 - 6,0 N/40mm aufweist.

6. Inkontinenzwegwerfwindel (2) nach einem oder mehreren der vorgenannten Ansprüche, wobei der Verbundwerkstoff (100) nach der in der Beschreibung dargelegten Prüfmethode bei erstmaliger Dehnung in der Querrichtung (10) um 200% eine Rückstellkraft von K200%,1 ≤ 14 N/25mm, insbesondere von K200%,1 ≤ 12 N/25mm, weiter insbesondere von K200%,1 ≤ 11 N/25mm, weiter insbesondere von K200%,1 ≥ 6 N/25mm, weiter insbesondere von K200%,1 ≥ 8 N/25mm aufweist, und weiter insbesondere nach der in der Beschreibung dargelegten Prüfmethode bei zweiter Dehnung in der Querrichtung (10) um 200% eine Rückstellkraft von K200%,2 ≤ 10 N/25mm, insbesondere ≤ 8 N/25mm, und weiter insbesondere ≥ 3,5 N/25mm, insbesondere ≥ 5,5 N/25mm aufweist.

7. Inkontinenzwegwerfwindel (2) nach einem oder mehreren der vorgenannten Ansprüche, wobei der Verbundwerkstoff (100) nach der in der Beschreibung dargelegten Prüfmethode einen dritten Kraftabfall DeltaK3 = K80%,1,200% - K80%,2, 200% von ≤ 6 N/25mm, insbesondere von ≤ 5 N/25mm, insbesondere von ≤ 4 N/25mm, insbesondere von ≥ 0,5 N/25mm aufweist, und weiter insbesondere nach der in der Beschreibung dargelegten Prüfmethode einen vierten Kraftabfall DeltaK4 = K200%,2 - K20%,2,200% von ≤ 9 N/25mm, insbesondere von ≤ 8 N/25mm, insbesondere von ≤ 7 N/25mm, insbesondere von ≥ 1,5 N/25mm, insbesondere von ≥ 2,5 N/25mm, insbesondere von ≥ 3,5 N/25mm aufweist.

8. Inkontinenzwegwerfwindel (2) nach einem oder mehreren der vorgenannten Ansprüche, wobei die elastische Folienlage (101) eine Polymermischung umfasst oder daraus besteht.

9. Inkontinenzwegwerfwindel (2) nach einem oder mehreren der vorgenannten Ansprüche, wobei die elastische Folienlage (101) weniger als 10 Gew.-% medizinische Weißöle, insbesondere weniger als 5 Gew.-% medizinische Weißöle, insbesondere weniger als 3 Gew.-%, weiter insbesondere keine medizinischen Weißöle enthält.

10. Inkontinenzwegwerfwindel (2) nach einem oder mehreren der vorgenannten Ansprüche, wobei die elastische Folienlage (101) weniger als 10 Gew.-% technische Weißöle, insbesondere weniger als 5 Gew.-%, weiter insbesondere weniger als 3 Gew.-%, weiter insbesondere keine technischen Weißöle enthält.

11. Inkontinenzwegwerfwindel (2) nach einem oder mehreren der vorgenannten Ansprüche, wobei die elastische Folienlage (101) weniger als 10 Gew.-%, insbesondere weniger als 5 Gew.-%, insbesondere weniger als 3 Gew.-% insbesondere keine Mineralöle enthält.

12. Inkontinenzwegwerfwindel (2) nach einem oder mehreren der vorgenannten Ansprüche, wobei die elastische Folienlage (101) weniger als 10 Gew.-%, insbesondere weniger als 5 Gew.-%, insbesondere weniger als 3 Gew.-%,

weiter insbesondere keine Weichmacher enthält.

13. Inkontinenzwegwerfwindel (2) nach einem oder mehreren der vorgenannten Ansprüche, wobei die erste Vliesstofflage (102) vollflächig mit der Folienlage (101) verbunden ist.

14. Inkontinenzwegwerfwindel (2) nach einem oder mehreren der vorgenannten Ansprüche, wobei die erste Vliesstofflage (102) durch Extrusionslaminierung mit der Folienlage (101) verbunden ist.

15. Inkontinenzwegwerfwindel (2) nach einem oder mehreren der vorgenannten Ansprüche, wobei die erste Vliesstofflage (102) kein Punktbindemuster aufweist.

16. Inkontinenzwegwerfwindel (2) nach einem oder mehreren der vorgenannten Ansprüche, wobei die Folienlage (101) aus einer Polymermischung besteht oder diese umfasst, welche mindestens ein thermoplastisches Elastomer aus der Gruppe der Styrol/Isopren/Styrol-Triblock-Copolymere (SIS) enthält und ferner insbesondere mindestens ein Polymer, ausgewählt aus der Gruppe der olefinischen Polymere enthält, wobei die olefinischen Polymere ausgewählt sind aus der Gruppe der Polyethylene und Polypropylene und Copolymere von Ethylen und Propylen und thermoplastischen elastomeren Polyolefinen (TPOs),

17. Inkontinenzwegwerfwindel (2) nach Anspruch 16 **dadurch gekennzeichnet, dass** die olefinischen Polymere ausgewählt sind aus der Gruppe der mittels Metallocen-Katalyse hergestellten Polyethylene und Polypropylene und Copolymeren von Ethylen und Propylen.

18. Inkontinenzwegwerfwindel (2) nach Ansprüchen 16 oder 17 **dadurch gekennzeichnet, dass** die olefinischen Polymere ein Polypropylen oder ein Copolymer aus Ethylen und Propylen umfassen oder daraus bestehen.

19. Inkontinenzwegwerfwindel (2) nach einem oder mehreren der vorgenannten Ansprüche 16-18 wobei das SIS-Triblock-Copolymer der Folienlage (101) weniger als 10% oder weniger als 1% an Diblock-Bestandteilen enthält.

20. Inkontinenzwegwerfwindel (2) nach einem oder mehreren der vorgenannten Ansprüche, **dadurch gekennzeichnet, dass** ein Vliesstoff der ersten Vliesstofflage ausgewählt ist aus der Gruppe der mittels Wasserstrahlbindung gebundenen Vliesstoffe und/oder aus der Gruppe der einschichtigen Vliesstoffe.

21. Inkontinenzwegwerfwindel (2) nach einem oder mehreren der vorgenannten Ansprüche, **dadurch gekennzeichnet, dass** die Folienlage (101) eine Dicke von 100 Mikrometern oder weniger, oder im Bereich zwischen 20 und 100 Mikrometern, oder im Bereich zwischen 20 und 70 Mikrometern, aufweist.

22. Inkontinenzwegwerfwindel (2) nach einem oder mehreren der vorgenannten Ansprüche, **dadurch gekennzeichnet, dass** der Verbundwerkstoff (100) eine zweite Vliesstofflage (103) umfasst, die direkt mit der elastischen Folienlage (101) verbunden ist, wobei die zweite Vliesstofflage (103) oberhalb und die erste Vliesstofflage (102) unterhalb der elastischen Folienlage (101) angeordnet ist.

23. Inkontinenzwegwerfwindel (2) nach einem oder mehreren der vorgenannten Ansprüche, wobei der Verbundwerkstoff (100) nach der in der Beschreibung dargelegten Prüfmethode beständig gegenüber Mandelöl oder Rapsöl oder Paraffinöl ist.

24. Inkontinenzwegwerfwindel (2) nach einem oder mehreren der vorgenannten Ansprüche, wobei an den Vorderbereich (12) keine Seitenabschnitte angefügt sind, sondern die vorderen seitlichen Längsränder des Hauptteils (4) einen frei endenden Längsrand der Inkontinenzwegwerfwindel (2) bilden, und wobei weiter insbesondere die hinteren Seitenabschnitte (22) im Bereich ihres in der Querrichtung (10) freien Endes jeweils wenigstens ein Verschlussmittel (28) aufweisen, wobei die hinteren Seitenabschnitte (22) zum Anlegen und Schließen der Inkontinenzwegwerfwindel (2) an einen Benutzer jeweils entlang einer Umfangsrichtung um den Körper des Benutzers herumlegbar und in überlappende Anordnung mit einer Außenseite des Vorderbereiches (12) bringbar sind, an der sie über das jeweilige Verschlussmittel (28) jeweils lösbar anhaftbar sind.

## Claims

1. Disposable incontinence nappy (2) having a longitudinal direction (8) and a transverse direction (10) and a main part (4), having an absorbent body (6), comprising a front region (12) with front lateral longitudinal edges and a back region (16) with rear lateral longitudinal edges (18) and a crotch region arranged therebetween which comes to rest between the legs of a user, and comprising rear side sections (22) which are joined on both sides to the back region (16) and which extend in the transverse direction (10) beyond the lateral rear longitudinal edges (18) of the main part (4), and wherein the rear side sections (22) have a component which is elastic in the transverse direction (10), wherein the elastic component comprises an elastic composite material (100), wherein the composite material (100) comprises an elastic film ply (101) and a first nonwoven ply (102) connected to the elastic film ply (101), wherein the film ply (101) has a single-layer structure, wherein the first nonwoven ply (102) is connected directly to the elastic film ply (101), wherein the composite material (100) exhibits a restoring force of K140%.1 ≤ 12 N/25 mm when stretched in the transverse direction (10) by 140% for the first time and wherein the composite material (100) exhibits a glyceryl trioleate resistance RGly150% of ≥ 50% and wherein K140%.1 and RGly150% can be ascertained according to the test method outlined in each case in the description.

2.  Disposable incontinence nappy (2) according to Claim 1, wherein, according to the test method outlined in the description, the composite material (100) exhibits a restoring force of K140%.1 ≤ 10 N/25 mm, in particular ≥ 2 N/25 mm, more particularly ≥ 4 N/25 mm, when stretched in the transverse direction (10) by 140% for the first time, and more particularly, according to the test method outlined in the description, exhibits a restoring force of K140%.2 ≤ 10 N/25 mm, in particular ≤ 8 N/25 mm, more particularly ≥ 1.5 N/25 mm, more particularly ≥ 3.5 N/25 mm, when stretched in the transverse direction (10) by 140% for the second time.

3.  Disposable incontinence nappy (2) according to one or more of the preceding claims, wherein, according to the test method outlined in the description, the composite material (100) exhibits a first drop in force DeltaK1 = K80%.1, 140% - K80%.2, 140% of ≤ 6 N/25 mm, in particular of ≤ 5 N/25 mm, in particular of ≤ 4 N/25 mm, in particular of ≥ 0.5 N/25 mm, and more particularly, according to the test method outlined in the description, exhibits a second drop in force DeltaK2 = K140%.2 - K20%.2, 140% of ≤ 9 N/25 mm, in particular of ≤ 8 N/25 mm, in particular of ≤ 7 N/25 mm, in particular of ≥ 1.5 N/25 mm, in particular of ≥ 2.5 N/25 mm, in particular of ≥ 3.5 N.

4.  Disposable incontinence nappy (2) according to one or more of the preceding claims, wherein, according to the test method outlined in the description, the composite material (100) exhibits a glyceryl trioleate resistance RGly150% of ≥ 55%, more particularly of ≥ 60%, more particularly of ≥ 65%, more particularly of ≤ 95%, more particularly of ≤ 90%, more particularly of ≤ 85%.

5.  Disposable incontinence nappy (2) according to one or more of the preceding claims, wherein, according to the test method outlined in the description, the composite material (100) exhibits a restoring force KGly150% of 2.5-12.5 N/40 mm, in particular of 5.5-10.5 N/40 mm, and more particularly, according to the test method outlined in the description, exhibits a restoring force KGly140% of 1.5-7.0 N/40 mm, in particular of 3.0-6.0 N/40 mm.

6.  Disposable incontinence nappy (2) according to one or more of the preceding claims, wherein, according to the test method outlined in the description, the composite material (100) exhibits a restoring force of K200%.1 ≤ 14 N/25 mm, in particular of K200%.1 ≤ 12 N/25 mm, more particularly of K200%.1 ≤ 11 N/25 mm, more particularly of K200%.1 ≥ 6 N/25 mm, more particularly of K200%.1 ≥ 8 N/25 mm, when stretched in the transverse direction (10) by 200% for the first time, and more particularly, according to the test method outlined in the description, exhibits a restoring force of K200%.2 ≤ 10 N/25 mm, in particular ≤ 8 N/25 mm, and more particularly ≥ 3.5 N/25 mm, in particular ≥ 5.5 N/25 mm, when stretched in the transverse direction (10) by 200% for the second time.

7.  Disposable incontinence nappy (2) according to one or more of the preceding claims, wherein, according to the test method outlined in the description, the composite material (100) exhibits a third drop in force DeltaK3 = K80%.1, 200% - K80%.2, 200% of ≤ 6 N/25 mm, in particular of ≤ 5 N/25 mm, in particular of ≤ 4 N/25 mm, in particular of ≥ 0.5 N/25 mm, and more particularly, according to the test method outlined in the description, exhibits a fourth drop in force DeltaK4 = K200%.2 - K20%.2, 200% of ≤ 9 N/25 mm, in particular of ≤ 8 N/25 mm, in particular of ≤ 7 N/25 mm, in particular of ≥ 1.5 N/25 mm, in particular of ≥ 2.5 N/25 mm, in particular of ≥ 3.5 N/25 mm.

8.  Disposable incontinence nappy (2) according to one or more of the preceding claims, wherein the elastic film ply (101) comprises or consists of a polymer mixture.

9.  Disposable incontinence nappy (2) according to one or more of the preceding claims, wherein the elastic film ply (101) contains less than 10% by weight of medicinal white oils, in particular less than 5% by weight of medicinal white oils, in particular less than 3% by weight, more particularly no medicinal white oils.

10. Disposable incontinence nappy (2) according to one or more of the preceding claims, wherein the elastic film ply (101) contains less than 10% by weight of technical white oils, in particular less than 5% by weight, more particularly less than 3% by weight, more particularly no technical white oils.

11. Disposable incontinence nappy (2) according to one or more of the preceding claims, wherein the elastic film ply (101) contains less than 10% by weight, in particular less than 5% by weight, in particular less than 3% by weight, in particular no mineral oils.

12. Disposable incontinence nappy (2) according to one or more of the preceding claims, wherein the elastic film ply (101) contains less than 10% by weight, in particular less than 5% by weight, in particular less than 3% by weight, more particularly no plasticizers.

13. Disposable incontinence nappy (2) according to one or more of the preceding claims, wherein the first nonwoven ply (102) is connected to the elastic film ply (101) in a full-area manner.

14. Disposable incontinence nappy (2) according to one or more of the preceding claims, wherein the first nonwoven ply (102) is connected to the film ply (101) by extrusion lamination.

15. Disposable incontinence nappy (2) according to one or more of the preceding claims, wherein the first nonwoven ply (102) does not have a punctiform bonding pattern.

16. Disposable incontinence nappy (2) according to one or more of the preceding claims, wherein the film ply (101) consists of or comprises a polymer mixture which contains at least one thermoplastic elastomer from the group of styrene-isoprene-styrene (SIS) triblock copolymers and also contains in particular at least one polymer selected from the group of olefinic polymers, wherein the olefinic polymers are selected from the group of polyethylenes and polypropylenes and copolymers of ethylene and propylene and thermoplastic elastomeric polyolefins (TPOs) .

17. Disposable incontinence nappy (2) according to Claim 16, **characterized in that** the olefinic polymers are selected from the group of metallocene-catalysis-prepared polyethylenes and polypropylenes and copolymers of ethylene and propylene.

18. Disposable incontinence nappy (2) according to Claim 16 or 17, **characterized in that** the olefinic polymers comprise or consist of a polypropylene or a copolymer of ethylene and propylene.

19. Disposable incontinence nappy (2) according to one or more of the preceding Claims 16-18, wherein the SIS triblock copolymer of the film ply (101) contains less than 10% by weight or less than 1% by weight of diblock constituents.

20. Disposable incontinence nappy (2) according to one or more of the preceding claims, **characterized in that** a nonwoven of the first nonwoven ply is selected from the group of nonwovens bonded by water jet bonding and/or from the group of single-layer nonwovens.

21. Disposable incontinence nappy (2) according to one or more of the preceding claims, **characterized in that** the film ply (101) has a thickness of 100 micrometres or less, or in the range between 20 and 100 micrometres, or in the range between 20 and 70 micrometres.

22. Disposable incontinence nappy (2) according to one or more of the preceding claims, **characterized in that** the composite material (100) comprises a second nonwoven ply (103) which is connected directly to the elastic film ply (101), wherein the second nonwoven ply (103) is arranged above the elastic film ply (101) and the first nonwoven ply (102) is arranged below said elastic film ply.

23. Disposable incontinence nappy (2) according to one or more of the preceding claims, wherein, according to the test method outlined in the description, the composite material (100) is resistant to almond oil or rapeseed oil or paraffin oil.

24. Disposable incontinence nappy (2) according to one or more of the preceding claims, wherein no side sections are joined to the front region (12), but rather the front lateral longitudinal edges of the main part (4) form a free-ending longitudinal edge of the disposable incontinence nappy (2), and wherein more particularly the rear side sections (22) each have, in the region of their end which is free in the transverse direction (10), at least one closure means (28), wherein, for the purpose of applying the disposable incontinence nappy (2) to a user and closing said nappy, the rear side sections (22) can each be wrapped around the body of the user in a circumferential direction and brought into overlapping arrangement with an outer side of the front region (12), to which they can each be releasably attached by way of the respective closure means (28).

**Revendications**

1. Couche d'incontinence jetable (2) présentant une direction longitudinale (8) et une direction transversale (10) et comprenant une partie principale (4), qui est pourvue d'un corps absorbant (6) et qui comprend une zone avant (12) munie de bords longitudinaux latéraux avant et une zone arrière (16), qui comprend des bords longitudinaux arrière (18), et une zone d'entrejambe disposée entre lesdites zones précédentes et qui vient se placer entre les jambes d'un utilisateur et comprenant des portions latérales arrière (22) qui sont fixées de part et d'autre de la zone arrière (16) et qui s'étendent dans la direction transversale (10) au-delà des bords longitudinaux latéraux arrière (18) de la partie principale (4), et les portions latérales arrière (22) présentant une partie constituante élastique dans la direction transversale (10), la partie constituante élastique comprenant un matériau composite élastique (100), le matériau composite (100) comprenant une couche de film élastique (101) et une première couche de non-tissé (102) reliée à la couche de film élastique (101), la couche de film (101) ayant une structure monocouche, la première couche de non-tissé (102) étant reliée directement à la couche de film élastique (101), le matériau composite (100) ayant une force de rappel de K140 %,1 ≤ 12 N/25 mm lorsqu'il est étiré pour la première fois de 140 % dans la direction transversale (10) et le matériau composite (100) présentant une résistance au trioléate de glycéryle RGly150 % ≥ 50 % et K140 %,1 et RGly150 % devant être déterminés selon la méthode d'essai décrite dans la description.

2. Couche d'incontinence jetable (2) selon la revendication 1, le matériau composite (100) ayant une force de rappel de K140 %,1 ≤ 10 N/25 mm, en particulier ≥ 2 N/25 mm, plus particulièrement ≥ 4 N/25 mm, selon la méthode d'essai présentée dans la description lorsqu'il est étiré pour la première fois de 140 % dans la direction transversale (10) et plus particulièrement une force de rappel de K140 %,2 ≤ 10 N/25 mm, notamment ≤ 8 N/25 mm, plus particulièrement ≥ 1,5 N/25 mm, plus particulièrement > 3,5 N/25 mm, selon la méthode d'essai présentée dans la description lorsqu'il est étiré pour la deuxième fois de 140 % dans la direction transversale (10).

3. Couche d'incontinence jetable (2) selon une ou plusieurs des revendications précédentes, le matériau composite (100) présentant une première chute de force DeltaK1 = K80 %,1, 140 % à K80 %,2, 140 % ≤ 6 N/25 mm, en particulier ≤ 5 N/25 mm, en particulier ≤ 4 N/25 mm, notamment ≥ 0,5 N/25 mm et plus particulièrement selon la méthode d'essai présentée dans la description une deuxième chute de force DeltaK2 = K140%,2 à K20%,2, 140 % ≤ 9 N/25 mm, notamment ≤ 8 N/25 mm, notamment ≤ 7 N/25 mm, notamment ≥ 1,5 N/25 mm, notamment ≥ 2,5 N/25 mm, notamment ≥ 3,5 N.

4. Couche d'incontinence jetable (2) selon une ou plusieurs des revendications précédentes, le matériau composite (100) présentant selon la méthode d'essai présentée dans la description une résistance au trioléate de glycéryle RGly150 % ≥ 55 %, plus particulièrement ≥ 60 %, plus particulièrement ≥ 65 %, plus particulièrement ≤ 95 %, plus particulièrement ≤ 90 %, plus particulièrement ≤ 85 %.

5. Couche d'incontinence jetable (2) selon une ou plusieurs des revendications précédentes, le matériau composite (100) présentant une force de rappel KGly150 % de 2,5 à 12,5 N/40 mm, en particulier de 5,5 à 10,5 N/40 mm selon la méthode d'essai décrite dans la description, et plus particulièrement selon la méthode d'essai présentée dans la description une force de rappel Kgly140 % de 1,5 à 7,0 N/40 mm, en particulier de 3,0 à 6,0 N/40 mm.

6. Couche d'incontinence jetable (2) selon une ou plusieurs des revendications précédentes, le matériau composite (100) présentant, selon la méthode d'essai présentée dans la description lorsqu'il est étiré pour la première fois de 200 % dans la direction transversale (10), une force de rappel de K200 %,1 ≤ 14 N/25 mm, en particulier de K200 %,1 ≤ 12 N /25 mm, plus particulièrement de K200 %,1 ≤ 11 N/25 mm, plus particulièrement de K200%,1 ≥ 6 N/25 mm, plus particulièrement de K200%,1 ≥ 8 N/25 mm, et plus particulièrement selon la méthode d'essai présentée dans la description avec un deuxième allongement de 200 % dans le sens transversal (10) une force de rappel de K200%,2 ≤ 10 N/25 mm, notamment ≤ 8 N/25 mm, et plus particulièrement ≥ 3,5 N/25mm, notamment ≥ 5,5 N/25 mm.

7. Couche d'incontinence jetable (2) selon une ou plusieurs des revendications précédentes, le matériau composite (100) présentant une troisième chute de force DeltaK3 = K80 %,1,200 % à K80 %,2,200 % ≤ 6 N/25 mm, en particulier ≤ 5 N/25 mm, en particulier ≤ 4 N/25 mm, en notamment ≥ 0,5 N/25 mm, et plus particulièrement selon la méthode d'essai présentée dans la description une quatrième chute de force DeltaK4 = K200 %,2 à K20 %,2,200 % ≤ 9 N/25 mm, notamment ≤ 8 N/25 mm, notamment ≤ 7 N/25 mm, notamment ≥ 1,5 N/25 mm, notamment ≥ 2,5 N/25 mm, notamment ≥ 3,5 N/25 mm.

8. Couche d'incontinence jetable (2) selon une ou plusieurs des revendications précédentes, la couche de film élastique (101) étant ou comprenant un mélange de polymères.

9. Couche d'incontinence jetable (2) selon une ou plusieurs des revendications précédentes, la couche de film élastique (101) contenant moins de 10 % en poids, en particulier moins de 5 % en poids, en particulier moins de 3 % en poids d'huiles blanches médicinales, plus particulièrement ne contenant pas d'huiles blanches médicinales.

10. Couche d'incontinence jetable (2) selon une ou plusieurs des revendications précédentes, la couche de film élastique (101) contenant moins de 10 % en poids, en particulier moins de 5 % en poids, plus particulièrement moins de 3 % en poids, d'huiles blanches techniques, plus particulièrement ne contenant pas d'huiles blanches techniques.

11. Couche d'incontinence jetable (2) selon une ou plusieurs des revendications précédentes, la couche de film élastique (101) contenant moins de 10 % en poids, en particulier moins de 5 % en poids, en particulier moins de 3 % en poids, d'huiles minérales, en particulier ne contenant pas d'huiles minérales.

12. Couche d'incontinence jetable (2) selon une ou plusieurs des revendications précédentes, la couche de film élastique (101) contenant moins de 10 % en poids, en particulier moins de 5 % en poids, en particulier moins de 3 % en poids, de plastifiant et en particulier ne contenant aucun plastifiant.

13. Couche d'incontinence jetable (2) selon une ou plusieurs des revendications précédentes, la première couche de non-tissé (102) étant reliée sur toute sa surface à la couche de film (101).

14. Couche d'incontinence jetable (2) selon une ou plusieurs des revendications précédentes, la première couche de non-tissé (102) étant reliée à la couche de film (101) par laminage par extrusion.

15. Couche d'incontinence jetable (2) selon une ou plusieurs des revendications précédentes, la première couche de non-tissé (102) ne comportant pas de motif de liaison ponctuelle.

16. Couche d'incontinence jetable (2) selon une ou plusieurs des revendications précédentes, la couche de film (101) étant ou comprenant un mélange de polymères qui contient au moins un élastomère thermoplastique du groupe des copolymères à trois blocs styrène/isoprène/styrène (SIS) et en outre notamment au moins un polymère choisi dans le groupe des polymères oléfiniques, les polymères oléfiniques étant choisis dans le groupe comprenant les polyéthylènes et les polypropylènes et des copolymères d'éthylène et de propylène et des élastomères thermoplastiques polyoléfines (TPO).

17. Couche d'incontinence jetable (2) selon la revendication 16, **caractérisée en ce que** les polymères oléfiniques sont choisis dans le groupe comprenant les polyéthylènes et les polypropylènes et les copolymères d'éthylène et de propylène produits par catalyse au métallocène.

18. Couche d'incontinence jetable (2) selon les revendications 16 ou 17, **caractérisée en ce que** les polymères oléfiniques sont ou comprennent un polypropylène ou un copolymère d'éthylène et de propylène.

19. Couche d'incontinence jetable (2) selon une ou plusieurs des revendications précédentes 16 à 18, le copolymère à trois blocs SIS de la couche de film (101) contenant moins de 10 % ou moins de 1 % de constituants à deux blocs.

20. Couche d'incontinence jetable (2) selon une ou plusieurs des revendications précédentes, **caractérisée en ce qu'**un non-tissé de la première couche de non-tissé est choisi dans le groupe des non-tissés liés au moyen d'une liaison par jet d'eau et/ou dans le groupe des non-tissés monocouche.

21. Couche d'incontinence jetable (2) selon une ou plusieurs des revendications précédentes, **caractérisée en ce que** la couche de film (101) a une épaisseur de 100 micromètres ou moins, ou dans la gamme comprise entre 20 et 100 micromètres, ou dans la gamme comprise entre 20 et 70 micromètres.

22. Couche d'incontinence jetable (2) selon une ou plusieurs des revendications précédentes, **caractérisée en ce que** le matériau composite (100) comprend une deuxième couche de non-tissé (103) qui est reliée directement à la couche de film élastique (101), la deuxième couche de non-tissé (103) étant disposée au-dessus de la couche de film élastique (101) et la première couche de non-tissé (102) étant disposée au-dessous de la couche de film élastique (101).

23. Couche d'incontinence jetable (2) selon une ou plusieurs des revendications précédentes, le matériau composite (100) étant résistant à l'huile d'amande ou à l'huile de colza ou à l'huile de paraffine selon la méthode d'essai présentée dans la description.

24. Couche d'incontinence jetable (2) selon une ou plusieurs des revendications précédentes, aucune portion latérale n'étant fixée à la zone avant (12), mais les bords longitudinaux latéraux avant de la partie principale (4) formant un bord longitudinal à extrémité libre de la couche d'incontinence jetable (2), et en particulier les portions latérales arrière (22) comportant chacune au moins un moyen de fermeture (28) au niveau de leur extrémité libre dans la direction transversale (10), les portions latérales arrière (22) pouvant être disposées autour du corps d'un utilisateur le long d'une direction périphérique et être placées en chevauchement avec un côté extérieur de la zone avant (12), sur lequel elles peuvent être fixées de manière amovible par le biais du moyen de fermeture respectif (28), afin d'appliquer et de fermer la couche d'incontinence jetable (2) sur l'utilisateur.

Fig. 1

Fig. 2

Fig. 3

Fig. 4a

Fig. 4b

Fig. 4c

Fig. 5

Fig. 6

Fig. 7

Fig. 8

Fig. 9

Fig. 10a

Fig. 10b

EP 3 758 660 B1

Figure 11

Figure 12

Figure 13

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007042084 A1
- WO 2017114695 A1
- WO 2004060665 A
- EP 3187333 A
- US 20040121692 A1

**In der Beschreibung aufgeführte Nicht-Patentliteratur**

- *CHEMICAL ABSTRACTS,* 122-32-7
- *CHEMICAL ABSTRACTS,* 90320-37-9
- *CHEMICAL ABSTRACTS,* 8002-13-9